# FASCICULE DE BREVET EUROPEEN

(11) **EP 2 147 915 B1**
(45) Date de publication et mention de la délivrance du brevet: **15.09.2010**
(21) Numéro de dépôt: 09290547.0
(22) Date de dépôt: 08.07.2009
(51) Int. Cl.: C07D 285/28, A61K 31/549, A61P 25/00

(54) **Nouveaux dérivés de benzothiadiazines cycloalkylées, leur procédé de préparation et les compositions pharmaceutiques qui les contiennent**
Neue Cycloalkyl-Benzothiadiazin-Derivate, ihr Herstellungsverfahren und die pharmazeutischen Zusammensetzungen, die sie enthalten
New cycloalkylated benzothiadiazine derivatives, method of preparing same and pharmaceutical compositions containing them

(30) Priorité: 09.07.2008 FR 0803898
(43) Date de publication de la demande: 27.01.2010
(73) Titulaire: Les Laboratoires Servier, 92284 Suresnes Cedex (FR)
(72) Inventeur: Francotte, Pierre, 4430 Ans (BE); De Tullio, Pascal, 4020 Liege (BE); Pirotte, Bernard, 4680 Oupeye (BE); Danober, Laurence, 78360 Montesson (FR); Lestage, Pierre, 78170 La Celle Saint Cloud (FR); Caignard, Daniel-Henri, 95000 Boisemont (FR)

(56) Documents cités:
- WO-A-03/031422
- WHITEHEAD, CALVERT W. ET AL: "Diuretics. VI. 1,2,4-Benzothiadiazine 1,1-dioxides substituted at 2,3,4- and 7-N-sulfamoyl positions" JOURNAL OF ORGANIC CHEMISTRY , 27, 951-6 CODEN: JOCEAH; ISSN: 0022-3263, 1962, XP002515880
- DATABASE CAPLUS [Online] CHEMICAL ABSTRACTS SERVICE, COLUMBUS, OHIO, US; WHEATLEY, DAVID ET AL: "Hypertension. Results of treatment [with various drugs]" XP002515882 extrait de STN Database accession no. 1964:12490 & PROCEEDINGS OF THE ROYAL SOCIETY OF MEDICINE , 56(5), 400-6 CODEN: PRSMA4; ISSN: 0035-9157, 1963,
- PHILLIPS, DEAN ET AL: "5'-Alkyl-benzothiadiazides: A New Subgroup of AMPA Receptor Modulators with Improved Affinity" BIOORGANIC & MEDICINAL CHEMISTRY , 10(5), 1229-1248 CODEN: BMECEP; ISSN: 0968-0896, 2002, XP002515881

## Description

La présente invention concerne de nouveaux dérivés de benzothiadiazines cycloalkylées, leur procédé de préparation, les compositions pharmaceutiques qui les contiennent ainsi que leur utilisation en tant que modulateurs allostériques positifs des récepteurs AMPA.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral Reviews, 1992, 16, 13-24 ; Progress in Neurobiology, 1992, 39, 517-545).

Par ailleurs, certains travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits comme modulant positivement les récepteurs AMPA des cellules neuronales (J. Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit, entre autres, certains dérivés de benzothiadiazine en tant que modulateurs des récepteurs AMPA.

Les dérivés de benzothiadiazine, objets de la présente invention, outre le fait qu'ils soient nouveaux, présentent, de manière surprenante, des activités pharmacologiques pour le récepteur AMPA nettement supérieures à celles des composés de structures proches décrits dans l'Art Antérieur.

Plus spécifiquement, la présente invention concerne les composés de formule (I) : 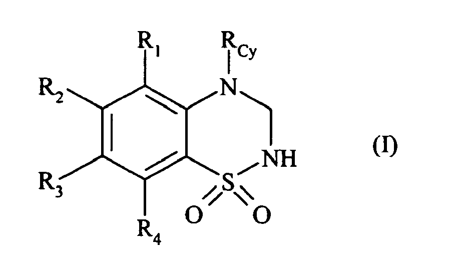 dans laquelle :
➢ R_{Cy} représente :
   ■ un groupement cycloalkyle (C₃-C₈) non-substitué ou substitué par un ou plusieurs groupements, identiques ou différents, choisis parmi alkyle (C₁-C₆) linéaire ou ramifié non-substitué ou substitué par un ou plusieurs atomes d'halogène ; alkoxy (C₁-C₆) linéaire ou ramifié ; hydroxy ; et amino non-substitué ou substitué par un ou deux groupements alkyle (C₁-C₆) linéaire ou ramifié ;
   ■ ou un groupement cycloalkyle (C₃-C₈) alkyle (C₁-C₆) linéaire ou ramifié non-substitué ou substitué sur la partie cyclique par un ou plusieurs groupements, identiques ou différents, choisis parmi alkyle (C₁-C₆) linéaire ou ramifié non-substitué ou substitué par un ou plusieurs atomes d'halogène ; alkoxy (C₁-C₆) linéaire ou ramifié ; hydroxy ; et amino non-substitué ou substitué par un ou deux groupements alkyle (C₁-C₆) linéaire ou ramifié ;
➢ R₁, R₂, R₃ et R₄, identiques ou différents, représentent chacun un atome d'hydrogène ou d'halogène ou un groupement nitro ; cyano ; hydroxy ; thio ; alkyle (C₁-C₆) linéaire ou ramifié non-substitué ou substitué par un ou plusieurs atomes d'halogène ; cyanoalkyle (C₁-C₆) linéaire ou ramifié ; hydroxyalkyle (C₁-C₆) linéaire ou ramifié ; alkoxy (C₁-C₆) linéaire ou ramifié non-substitué ou substitué par un ou plusieurs atomes d'halogène ; alkylthio (C₁-C₆) linéaire ou ramifié ; carboxy ; alkoxycarbonyle (C₁-C₆) linéaire ou ramifié ; aryloxycarbonyle ; acyle (C₁-C₆) linéaire ou ramifié ; amino non-substitué ou substitué par un ou deux groupements alkyle (C₁-C₆) linéaire ou ramifié ou par un groupement acyle (C₁-C₆) linéaire ou ramifié ; aminocarbonyle non-substitué ou substitué par un ou deux groupements alkyle (C₁-C₆) linéaire ou ramifié ; arylaminocarbonyle ; ou alkylsulfonylamino (C₁-C₆) linéaire ou ramifié ;
   leurs énantiomères et leurs diastéréoisomères lorsqu'ils existent, ainsi que leurs sels d'addition avec un acide ou une base pharmaceutiquement acceptable.

On entend par « aryle », le groupement phényle non-substitué ou substitué par un ou plusieurs groupements, identiques ou différents, choisis parmi alkyle (C₁-C₆) linéaire ou ramifié non-substitué ou substitué par un ou plusieurs atomes d'halogène ; alkoxy (C₁-C₆) linéaire ou ramifié ; hydroxy ; et amino non-substitué ou substitué par un ou deux groupements alkyle (C₁-C₆) linéaire ou ramifié.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique.

Parmi les bases pharmaceutiquement acceptables, on peut citer à titre non limitatif l'hydroxyde de sodium, l'hydroxyde de potassium, la triéthylamine, la tertbutylamine.

Les groupements R_{Cy} préférés sont les groupements cycloalkyles (C₃-C₈), et plus particulièrement le groupement cyclopropyle. De manière avantageuse, le groupement cyclopropyle peut être substitué par un groupement alkyle (C₁-C₆) linéaire ou ramifié et, plus particulièrement, le groupement méthyle.

Les groupements R_{Cy} préférés sont les groupements cycloalkyle (C₃-C₈) alkyle (C₁-C₆) linéaire ou ramifié, et plus particulièrement le groupement cyclopropylméthyle.

Le groupement R₁ représente préférentiellement un atome d'hydrogène ou un atome d'halogène, et plus particulièrement l'atome de fluor.
Le groupement R₂ représente préférentiellement un atome d'hydrogène ; un atome d'halogène, et plus particulièrement l'atome de fluor, de chlore ou de brome ; le groupement cyano ; le groupement carboxy ; ou le groupement alkyle (C₁-C₆) linéaire ou ramifié, et plus particulièrement le groupement méthyle.
Le groupement R₃ représente préférentiellement un atome d'hydrogène ; un atome d'halogène, et plus particulièrement l'atome de fluor, de chlore ou de brome ; le groupement alkyle (C₁-C₆) linéaire ou ramifié, et plus particulièrement le groupement méthyle.
Le groupement R₄ représente un atome d'hydrogène ou un atome d'halogène, et plus particulièrement l'atome de fluor, de chlore ou de brome.

Sont plus particulièrement préférés les composés pour lesquels deux des groupements R₁. R₂, R₃ ou R₄ situés sur le noyau benzénique représentent un atome d'hydrogène, tandis que les deux autres substituants, identiques ou différents, représentent un groupement distinct d'un atome d'hydrogène, choisi préférentiellement parmi les atomes d'halogène, tels que l'atome de fluor, de chlore ou de brome.

De manière avantageuse, sont plus particulièrement préférés les composés pour lesquels R_{Cy} représente un groupement cyclopropyle et, deux des groupements R₁, R₂, R₃ ou R₄ situés sur le noyau benzénique représentent un atome d'hydrogène, tandis que les deux autres substituants, identiques ou différents, représentent un atome d'halogène, tels que l'atome de fluor, de chlore ou de brome.

Les composés préférés selon l'invention sont :
- le 6,7-dichloro-4-cyclopropyl-3,4-dihydro-2*H*-1,2,4-benzothiadiazine 1,1-dioxyde ;
- le 8-chloro-4-cyclopropyl-6-fluoro-3,4-dihydro-2*H*-1,2,4-benzothiadiazine 1,1-dioxyde ;
- le 8-bromo-4-cyclopropyl-6-fluoro-3,4-dihydro-2*H*-1,2,4-benzothiadiazine 1,1-dioxyde ;
- le 6-cyano-4-cyclopropyl-3,4-dihydro-2*H*-1,2,4-benzothiadiazine 1,1-dioxyde ;
- le 8-bromo-6-chloro-4-cyclopropyl-3,4-dihydro-2*H*-1,2,4-benzothiadiazine 1,1-dioxyde ;
- le 4-cyclopropyl-5-fluoro-3,4-dihydro-2*H*-1,2,4-benzothiadiazine 1,1-dioxyde ;
- le 8-bromo-4-cyclopropyl-3,4-dihydro-2*H*-1,2,4-benzothiadiazine 1,1-dioxyde ;
- le 4-cyclopropyl-5,7-difluoro-3,4-dihydro-2*H*-1,2,4-benzothiadiazine 1,1-dioxyde ;
- le 6-fluoro-4-cyclopropyl-3,4-dihydro-2*H*-1,2,4-benzothiadiazine 1,1-dioxyde ;
- le 8-chloro-4-cyclopropyl-7-fluoro-3,4-dihydro-2*H*-1,2,4-benzothiadiazine 1,1-dioxyde ;
- le 4-cyclopropyl-3,4-dihydro-6-méthyl-2*H*-1,2,4-benzothiadiazine 1,1-dioxyde ;
- le 4-cyclopropyl-8-fluoro-3,4-dihydro-2*H*-1,2,4-benzothiadiazine 1,1-dioxyde ;
- le 8-chloro-4-cyclopropyl-3,4-dihydro-2*H*-1,2,4-benzothiadiazine 1,1-dioxyde ;
- le 6-bromo-4-cyclopropyl-7-fluoro-3,4-dihydro-2*H*-1,2,4-benzothiadiazine 1,1-dioxyde ;
- le 6-bromo-4-cyclopropyl-3,4-dihydro-2*H*-1,2,4-benzothiadiazine 1,1-dioxyde ;
- le 4-cyclopropyl-6,8-difluoro-3,4-dihydro-2*H*-1,2,4-benzothiadiazine 1,1-dioxyde ;
- le 4-cyclopropyl-5,6-difluoro-3,4-dihydro-2*H*-1,2,4-benzothiadiazine 1,1-dioxyde ;
- le 4-cyclopropyl-3,4-dihydro-7-méthyl-2*H*-1,2,4-benzothiadiazine 1,1-dioxyde ;
- le 6-chloro-4-cyclopropyl-3,4-dihydro-2*H*-1,2,4-benzothiadiazine 1,1-dioxyde ;
- le 4-cyclopropyl-3,4-dihydro-6-iodo-2*H*-1,2,4-benzothiadiazine 1,1-dioxyde ;
- le 6,7-dichloro-4-(1-méthyl)cyclopropyl-3,4-dihydro-2*H*-1,2,4-benzothiadiazine 1,1-dioxyde ;
- le 4-cyclopropylméthyl-7-fluoro-3,4-dihydro-2*H*-1,2,4-benzothiadiazine 1,1-dioxyde ;
- le 6-carboxy-4-cyclopropyl-3,4-dihydro-2*H*-1,2,4-benzothiadiazine 1,1-dioxyde ;
- le 4-cyclopropyl-7,8-difluoro-3,4-dihydro-2*H*-1,2,4-benzothiadiazine 1,1-dioxyde ;
- le 8-bromo-6-cyano-4-cyclopropyl-3,4-dihydro-2*H*-1,2,4-benzothiadiazine 1,1-dioxyde ;
- et le 7-bromo-4-cyclopropyl-3,4-dihydro-2*H*-1,2,4-benzothiadiazine 1,1-dioxyde.

Les sels d'addition à un acide ou une base pharmaceutiquement acceptable des composés préférés de l'invention font partie intégrante de l'invention.

L'invention s'étend également au procédé de préparation des composés de formule (I) à partir du composé de formule (II) : 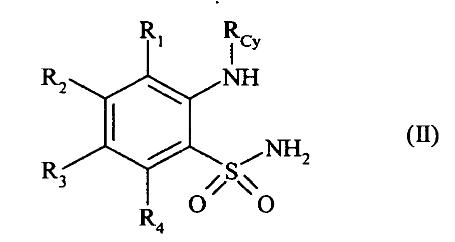 dans laquelle R_{Cy}, R₁, R₂, R₃ et R₄ sont tels que définis dans la formule (I),
que l'on cyclise en présence d'un composé de formule (III) :

H-C(OR₅)₃ (III)

dans laquelle R₅ représente un groupement alkyle (C₁-C₆) linéaire ou ramifié,
pour conduire au composé de formule (IV) : 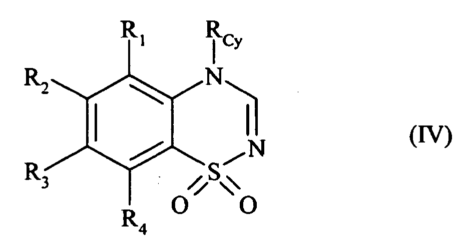 dans laquelle R_{Cy}, R₁, R₂, R₃ et R₄ sont tels que définis précédemment,
que l'on met en réaction avec un agent réducteur, pour conduire au composé de formule (I),
une variante dans la préparation des composés de formule (I) consistant, après réalisation de l'étape de réduction du composé de formule (IV), en l'utilisation des réactions classiques de chimie afin de modifier, dans un deuxième temps, les substituants du noyau benzénique,
composé de formule (I) qui peut être ensuite purifié selon une technique classique de séparation, que l'on transforme, si on le souhaite en ses sels d'addition à un acide ou à une base pharmaceutiquement acceptable et dont on sépare éventuellement les isomères, s'ils existent, selon une technique classique de séparation.

La présente invention concerne également le procédé de préparation des composés de formule (I) **caractérisé en ce que** l'on utilise comme produit de départ un composé de formule (V) : 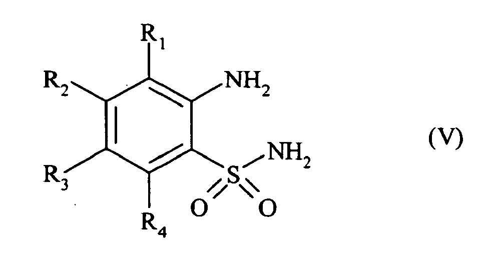 dans laquelle R₁, R₂, R₃ et R₄ sont tels que définis précédemment,
que l'on cyclise en présence d'un composé de formule (III) pour conduire au composé de formule (VI) : 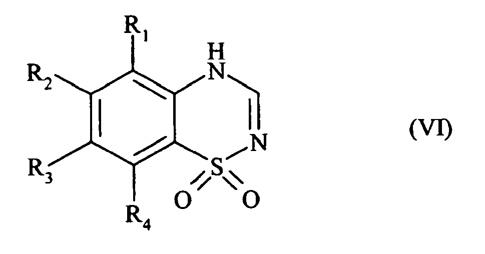 dans laquelle R₁, R₂, R₃ et R₄ sont tels que définis précédemment,
que l'on met en réaction avec un composé de formule (VII) :

Y-R_{Cy} (VII)

dans laquelle R_{Cy} est tel que défini précédemment et Y représente un groupement partant choisi parmi les atomes d'iode, de brome et les groupements tosylate, mésylate et triflate pour conduire au composé de formule (IV),
que l'on met en réaction avec un agent réducteur, pour conduire au composé de formule (I),
une variante dans la préparation des composés de formule (I) consistant, après réalisation de l'étape de réduction du composé de formule (IV), en l'utilisation des réactions classiques de chimie afin de modifier, dans un deuxième temps, les substituants du noyau benzénique,
composé de formule (I) qui peut être ensuite purifié selon une technique classique de séparation, que l'on transforme, si on le souhaite en ses sels d'addition à un acide ou à une base pharmaceutiquement acceptable et dont on sépare éventuellement les isomères, s'ils existent, selon une technique classique de séparation.

Les composés de formule (II) et de formule (V) sont aisément accessibles à l'homme du métier par des réactions de chimie classiques ou décrites dans la littérature.

Les composés de formule (IV) sont nouveaux et font également partie de l'invention à titre d'intermédiaires de synthèse des composés de formule (I).

Les composés de formule (I) selon l'invention présentent des propriétés activatrices des récepteurs AMPA qui les rendent utiles dans le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, et les suspensions buvables.

La posologie utile est adaptable selon la nature et la sévérité de l'affection, la voie d'administration ainsi que l'âge et le poids du patient. Cette posologie varie de 0,01 à 1000 mg par jour en une ou plusieurs prises.

Les exemples suivants illustrent l'invention mais ne la limitent en aucune façon.

Les produits de départ utilisés sont des produits connus ou préparés selon des modes préparatoires connus.

Les structures des composés décrits dans les exemples ont été déterminées selon les techniques spectrophotométriques usuelles (infrarouge, RMN, spectrométrie de masse).

### EXEMPLE 1 : 6,7-dichloro-4-cyclopropyl-3,4-dihydro-2H-1,2,4-benzothiadiazine 1,1-dioxyde

### Stade A : 4,5-dichloro-2-fluorobenzènesulfonamide

Dans un ballon de 500 mL, une portion d'acide acétique glacial (30 mL) est saturée pendant 30 minutes par un courant d'anhydride sulfureux gazeux. A cette solution est ajoutée une solution de chlorure cuivrique (1,5 g) dans l'eau (10 mL) (suspension A). La 4,5-dichloro-2-fluoroaniline (5 g) est dissoute dans un mélange d'acide acétique glacial (30 mL) et d'acide chlorhydrique concentré (15 mL). Cette solution est refroidie à -5 °C sur bain de glace et de sel. Ensuite, une solution de nitrite sodique (2,5 g) dans l'eau (10 mL) est ajoutée goutte à goutte sous agitation constante. Cette mixture est ajoutée lentement à la suspension A et est maintenue sous agitation sur bain de glace pendant 15 minutes. La mixture est ensuite versée sur un mélange d'eau (200 mL) et d'éther (200 mL). La phase éthérée est décantée et lavée à l'eau (100 mL). La phase organique est concentrée à siccité par distillation sous pression réduite et le résidu est redissous dans le dioxanne (25 mL). Cette solution est versée lentement sous agitation dans un mélange d'ammoniaque concentré (25 mL) et d'eau (10 mL) refroidi sur bain de glace. Après 30 minutes, la solution est évaporée à siccité par distillation sous pression réduite et le résidu obtenu est solubilisé dans le méthanol. La solution méthanolique est traitée au charbon adsorbant, filtrée et le filtrat évaporé à siccité. Le résidu est recristallisé dans un mélange méthanol-eau.
*Point de fusion : 144-145 °C*

### Stade B : 4,5-dichloro-2-cyclopropylaminobenzènesulfonamide

La solution de 4,5-dichloro-2-fluorobenzènesulfonamide (3 g) dans le dioxanne (30 mL) additionné de la cyclopropylamine (3 mL) est chauffée à 100-110°C en enceinte hermétique pendant 24 heures. Le solvant et l'excès d'amine sont éliminés par distillation sous pression réduite et le résidu est dissous dans le méthanol (20 mL). La solution méthanolique est refroidie sur bain de glace et additionnée d'eau (60 mL). Le précipité obtenu (produit du titre) est recueilli par filtration, lavé à l'eau et séché. Il est utilisé dans l'étape suivante sans autre purification.

### Stade C : 6,7-dichloro-4-cyclopropyl-4H-1,2,4-benzothiadiazine 1,1-dioxyde

Dans un ballon, le mélange de 4,5-dichloro-2-cyclopropylaminobenzènesulfonamide de l'étape précédente (2,5 g) et d'orthoformiate d'éthyle (25 mL) est chauffé en vase ouvert à 150 °C pendant 1 heure. La suspension obtenue est refroidie sur bain de glace et l'insoluble est recueilli par filtration, lavé à l'éther et séché. Le solide est redissous dans un mélange d'acétone et de méthanol à chaud et la solution chaude est traitée au charbon absorbant puis filtrée et concentrée à siccité. Le résidu est recristallisé dans le méthanol.
*Point de fusion : 260-262 °C*

### Stade D : 6,7-dichloro-4-cyclopropyl-3,4-dihydro-2H-1,2,4-benzothiadiazine 1,1-dioxyde

La solution de 6,7-dichloro-4-cyclopropyl-4*H*-1,2,4-benzothiadiazine 1,1-dioxyde de l'étape précédente (1,9 g) dans l'isopropanol (50 mL) est additionnée de NaBH₄ (1 g) finement broyé, puis chauffée pendant 5-10 minutes à 50-55 °C. Le solvant est éliminé par évaporation sous dépression. Le résidu est repris par de l'eau (50 mL) et amené à pH acide par addition de HCl 6 N. Le produit du titre est extrait par le dichlorométhane (3 x 30 mL). La phase organique est séchée sur MgSO₄ et filtrée. Le filtrat est évaporé à siccité et le résidu obtenu est recristallisé dans le méthanol/eau 1/1 (60 mL).
*Point de fusion : 174-176 °C*

### EXEMPLE 2 : 4-cyclopropyl-3,4-dihydro-7-méthyl-2H-1,2,4-benzothiadiazine 1,1-dioxyde

### Stade A : 2-fluoro-5-méthylbenzènesulfonamide

Le produit attendu est obtenu à partir de la 2-fluoro-5-méthylaniline selon le procédé décrit dans le stade A de l'Exemple 1.
*Point de fusion : 118-120 °C*

### Stade B : 2-cyclopropylamino-5-méthylbenzènesulfonamide

Le produit attendu est obtenu selon le procédé décrit (temps de chauffe de 72 heures) dans le stade B de l'Exemple 1.

### Stade C : 4-cyclopropyl-7-méthyl-4H-1,2,4-benzothiadiazine 1,1-dioxyde

Le produit attendu est obtenu selon le procédé décrit (temps de chauffe de 3 heures) dans le stade C de l'Exemple 1.
*Point de fusion : 192-195 °C*

### Stade D : 4-cyclopropyl-3,4-dihydro-7-méthyl-2H-1,2,4-benzothiadiazine 1,1-dioxyde

Le produit attendu est obtenu selon le procédé décrit dans le stade D de l'Exemple 1.
*Point de fusion : 144-145 °C*

### EXEMPLE 3 : 6-chloro-4-cyclopropyl-7-fluoro-3,4-dihydro-2H-1,2,4-benzothiadiazine 1,1-dioxyde

### Stade A : 4-chloro-2,5-difluorobenzènesulfonamide

Le produit attendu est obtenu à partir de la 4-chloro-2,5-difluoroaniline selon le procédé décrit dans le stade A de l'Exemple 1.
*Point de fusion : 154-157 °C*

### Stade B : 4-chloro-2-cyclopropylamino-5-fluorobenzènesulfonamide

Le produit attendu est obtenu selon le procédé décrit dans le stade B de l'Exemple 1.

### Stade C : 6-chloro-4-cyclopropyl-7-fluoro-4H-1,2,4-benzothiadiazine 1,1-dioxyde

Le produit attendu est obtenu selon le procédé décrit (temps de chauffe de 2 heures) dans le stade C de l'Exemple 1.
*Point de fusion : 205-206 °C*

### Stade D : 6-chloro-4-cyclopropyl-7-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 170-171 °C*

### EXEMPLE 4 : 4-cyclopropyl-7-fluoro-3,4-dihydro-2H-1,2,4-benzothiadiazine 1,1-dioxyde

### Stade A : 2,5-difluorobenzènesulfonamide

Le produit attendu est obtenu à partir de la 2,5-difluoroaniline selon le procédé décrit dans le stade A de l'Exemple 1.
*Point de fusion : 135-137 °C*

### Stade B : 2-cyc/opropylamino-5-fluorobenzènesulfonamide

Le produit attendu est obtenu selon le procédé décrit (temps de chauffe de 96 heures) dans le stade B de l'Exemple 1.

### Stade C : 4-cyclopropyl-7-fluoro-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 170-172 °C*

### Stade D : 4-cyclopropyl-7-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 163-164 °C*

### EXEMPLE 5 : 7-chloro-4-cyclopropyl-3,4-dihydro-2H-1,2,4-benzothiadiazine 1,1-dioxyde

### Stade A : 5-chloro-2-fluorobenzènesulfonamide

Le produit attendu est obtenu à partir de la 5-chloro-2-fluoroaniline selon le procédé décrit dans le stade A de l'Exemple 1.
*Point de fusion : 135-136 °C*

### Stade B : 5-chloro-2-cyclopropylaminobenzènesulfonamide

Le produit attendu est obtenu selon le procédé décrit dans le stade B de l'Exemple 1.

### Stade C : 7-chloro-4-cyclopropyl-4H-1,2,4-benzothiadiazine 1,1-dioxyde

Dans un autoclave scellé, le mélange de 2-cyclopropylaminobenzènesulfonamide de l'étape précédente (2,5 g) et d'orthoformiate d'éthyle (25 mL) est chauffé à 150 °C pendant 96 heures. La mixture est refroidie sur bain de glace et l'insoluble est recueilli par filtration, lavé à l'éther et séché. Le solide est redissous dans un mélange d'acétone et de méthanol à chaud et la solution chaude est traitée au charbon absorbant puis filtrée et concentrée à siccité. Le résidu est recristallisé dans le méthanol.
*Point de fusion : 229-231 °C*

### Stade D : 7-chloro-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 159-161 °C*

### EXEMPLE 6 : 4-cyclopropyl-6-fluoro-3,4-dihydro-2H-1,2,4-benzothiadiazine 1,1-dioxyde

### Stade A : 2,4-difluorobenzènesulfonamide

Le produit attendu est obtenu à partir de la 2,4-difluoroaniline selon le procédé décrit dans le stade A de l'Exemple 1.
*Point de fusion : 155-157 °C*

### Stade B : 2-cyclopropylamino-4-fluorobenzènesulfonamide

Le produit attendu est obtenu selon le procédé décrit dans le stade B de l'Exemple 1.

### Stade C : 4-cyclopropyl-6-fluoro-4H-1,2,4-benzothiadiazine 1,1-dioxyde

Le produit attendu est obtenu selon le procédé décrit (temps de chauffe de 10 heures à 130 °C) dans le stade C de l'Exemple 1.
*Point de fusion : 199-202 °C*

### Stade D : 4-cyclopropyl-6-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 164-165 °C*

### EXEMPLE 7 : 6-chloro-4-cyclopropyl-3,4-dihydro-2H-1,2,4-benzothiadiazine 1,1-dioxyde

### Stade A : 4-chloro-2-fluorobenzènesulfonamide

Le produit attendu est obtenu à partir de la 4-chloro-2-fluoroaniline selon le procédé décrit dans le stade A de l'Exemple 1.
*Point de fusion : 104-106* °C

### Stade B : 4-chloro-2-cyclopropylaminobenzènesulfonamide

Le produit attendu est obtenu selon le procédé décrit dans le stade B de l'Exemple 1.

### Stade C : 6-chloro-4-cyclopropyl-4H-1,2,4-benzothiadiazine 1,1-dioxyde

Le produit attendu est obtenu selon le procédé décrit (temps de chauffe de 5 heures à 130 °C) dans le stade C de l'Exemple 1.
*Point de fusion : 239-242 °C*

### Stade D : 6-chloro-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 174-176 °C*

### EXEMPLE 8 : 4-cyclopropyl-7-trifluorométhyl-3,4-dihydro-2H-1,2,4-benzothiadiazine 1,1-dioxyde

### Stade A : 2-fluoro-5-trifluorométhylbenzènesulfonamide

Le produit attendu est obtenu à partir de la 2-fluoro-5-trifluorométhylaniline selon le procédé décrit dans le stade A de l'Exemple 1.
*Point de fusion : 128-130 °C*

### Stade B : 2-cyclopropylamino-5-trifluorométhylbenzènesulfonamide

Le produit attendu est obtenu selon le procédé décrit dans le stade B de l'Exemple 1.

### Stade C : 4-cyclopropyl-7-trifluorométhyl-4H-1,2,4-benzothiadiazine 1,1-dioxyde

Le produit attendu est obtenu selon le procédé décrit (temps de chauffe de 5 heures à 130 °C) dans le stade C de l'Exemple 1.
*Point de fusion : 160-162 °C*

### Stade D : 4-cyclopropyl-7-trifluorométhyl-3,4-dihydro-2H-1,2,4-benzothiadiazine 1,1-dioxyde

Le produit attendu est obtenu selon le procédé décrit dans le stade D de l'Exemple 1.
*Point de fusion : 155-157 °C*

### EXEMPLE 9 : 4-cyclopropyl-3,4-dihydro-2H-1,2,4-benzothiadiazine 1,1-dioxyde

### Stade A : 2-fluorobenzènesulfonamide

Le produit attendu est obtenu à partir de la 2-fluoroaniline selon le procédé décrit dans le stade A de l'Exemple 1.
*Point de fusion : 160-162 °C*

### Stade B : 2-cyclopropylaminobenzènesulfonamide

Le produit attendu est obtenu selon le procédé décrit (temps de chauffe de 96 heures) dans le stade B de l'Exemple 1.

### Stade C : 4-cyclopropyl-4H-1,2,4-benzothiadiazine 1,1-dioxyde

Le produit attendu est obtenu selon le procédé décrit (temps de chauffe de 5 heures à 130 °C) dans le stade C de l'Exemple 1.
*Point de fusion : 191-194 °C*

### Stade D : 4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 165-166 °C*

### EXEMPLE 10 : 7-cyano-4-cyclopropyl-3,4-dihydro-2H-1,2,4-benzothiadiazine 1,1-dioxyde

### Stade A : 5-cyano-2-fluorobenzènesulfonamide

Le produit attendu est obtenu à partir de la 5-cyano-2-fluoroaniline selon le procédé décrit dans le stade A de l'Exemple 1.
*Point de fusion : 195-196 °C*

### Stade B : 5-cyano-2-cyclopropylaminobenzènesulfonamide

Le produit attendu est obtenu selon le procédé décrit dans le stade B de l'Exemple 1.

### Stade C : 7-cyano-4-cyclopropyl-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 268-270 °C*

### Stade D : 7-cyano-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 259-261 °C*

### EXEMPLE 11 : 6-bromo-4-cyclopropyl-7-trifluorométhyl-3,4-dihydro-2H-1,2,4-benzothiadiazine 1,1-dioxyde

### Stade A : 4-bromo-2-fluoro-5-trifluorométhylbenzènesulfonamide

Le produit attendu est obtenu à partir de la 4-bromo-2-fluoro-5-trifluorométhylaniline selon le procédé décrit dans le stade A de l'Exemple 1.

### Stade B : 4-bromo-2-cyclopropylamino-5-trifluorométhylbenzènesulfonamide

Le produit attendu est obtenu selon le procédé décrit dans le stade B de l'Exemple 1.

### Stade C : 6-bromo-4-cyclopropyl-7-trifluorométhyl-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 204-206 °C*

### Stade D : 6-bromo-4-cyclopropyl-7-trifluorométhyl-3,4-dihydro-2H-1,2,4-benzothiadiazine 1,1-dioxyde

Le produit attendu est obtenu selon le procédé décrit dans le stade D de l'Exemple 1.
*Point de fusion : 186-189 °C*

### EXEMPLE 12 : 6-bromo-5-chloro-4-cyclopropyl-3,4-dihydro-2H-1,2,4-benzothiadiazine 1,1-dioxyde

### Stade A : 4-bromo-3-chloro-2-fluorobenzènesulfonamide

Le produit attendu est obtenu à partir de la 4-bromo-3-chloro-2-fluoroaniline selon le procédé décrit dans le stade A de l'Exemple 1.

### Stade B : 4-bromo-3-chloro-2-cyclopropylaminobenzènesulfonamide

Le produit attendu est obtenu selon le procédé décrit dans le stade B de l'Exemple 1.

### Stade C : 6-bromo-5-chloro-4-cyclopropyl-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 199-201 °C*

### Stade D : 6-bromo-5-chloro-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 128-131* °C

### EXEMPLE 13 : 4-cyclopropyl-5-trifluorométhyl-3,4-dihydro-2H-1,2,4-benzothiadiazine 1,1-dioxyde

### Stade A : 2-fluoro-3-trifluorométhylbenzènesulfonamide

Le produit attendu est obtenu à partir de la 2-fluoro-3-trifluorométhylaniline selon le procédé décrit dans le stade A de l'Exemple 1.
*Point de fusion : 112-114 °C*

### Stade B : 2-cyc/opropylamino-3-trifluorométhylbenzènesulfonamide

Le produit attendu est obtenu selon le procédé décrit dans le stade B de l'Exemple 1.

### Stade C : 4-cyclopropyl-5-trifluorométhyl-4H-1,2,4-benzothiadiazine 1,1-dioxyde

Le produit attendu est obtenu selon le procédé décrit (temps de chauffe de 5 heures à 130 °C) dans le stade C de l'Exemple 1.
*Point de fusion : 180-182* °C

### Stade D : 4-cyclopropyl-5-trifluorométhyl-3,4-dihydro-2H-1,2,4-benzothiadiazine 1,1-dioxyde

Le produit attendu est obtenu selon le procédé décrit dans le stade D de l'Exemple 1.
*Point de fusion : 165-167 °C*

### EXEMPLE 14 : 8-chloro-4-cyclopropyl-6-fluoro-3,4-dihydro-2H-1,2,4-benzothiadiazine 1,1-dioxyde

### Stade A : 2-chloro-4,6-difluorobenzènesulfonamide

Le produit attendu est obtenu à partir de la 2-chloro-4,6-difluoroaniline selon le procédé décrit dans le stade A de l'Exemple 1.
*Point de fusion : 116-120 °C*

### Stade B : 2-chloro-6-cyclopropylamino-4-fluorobenzènesulfonamide

Le produit attendu est obtenu selon le procédé décrit dans le stade B de l'Exemple 1.

### Stade C : 8-chloro-4-cyclopropyl-6-fluoro-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point_de fusion : 252-255* °C

### Stade D : 8-chloro-4-cyclopropyl-6-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 190-192 °C*

### EXEMPLE 15 : 8-chloro-4-cyclopropyl-3,4-dihydro-7-méthyl-2H-1,2,4-benzothiadiazine 1,1-dioxyde

### Stade A : 2-chloro-6-fluoro-3-méthylbenzènesulfonamide

Le produit attendu est obtenu à partir de la 2-chloro-6-fluoro-3-méthylaniline selon le procédé décrit dans le stade A de l'Exemple 1.

### Stade B : 2-chloro-6-cyclopropylamino-3-méthylbenzènesulfonamide

Le produit attendu est obtenu selon le procédé décrit dans le stade B de l'Exemple 1.

### Stade C : 8-chloro-4-cyclopropyl-7-méthyl-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 218-223 °C*

### Stade D : 8-chloro-4-cyclopropyl-3,4-dihydro-7-méthyl-2H-1,2,4-benzothiadiazine 1,1-dioxyde

Le produit attendu est obtenu selon le procédé décrit dans le stade D de l'Exemple 1.
*Point_de fusion : 230-232 °C*

### EXEMPLE 16 : 6-cyano-4-cyclopropyl-3,4-dihydro-2H-1,2,4-benzothiadiazine 1,1-dioxyde

### Stade A : 4-cyano-2-fluorobenzènesulfonamide

Le produit attendu est obtenu à partir de la 4-cyano-2-fluoroaniline selon le procédé décrit dans le stade A de l'Exemple 1.
*Point de fusion : 149-152 °C*

### Stade B : 4-cyano-2-cyclopropylaminobenzènesulfonamide

Le produit attendu est obtenu selon le procédé décrit dans le stade B de l'Exemple 1.

### Stade C : 6-cyano-4-cyclopropyl-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 273-276 °C*

### Stade D : 6-cyano-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 178-180 °C*

### EXEMPLE 17 : 8-bromo-4-cyclopropyl-6-fluoro-3,4-dihydro-2H-1,2,4-benzothiadiazine 1,1-dioxyde

### Stade A : 2-bromo-4,6-difluorobenzènesulfonamide

Le produit attendu est obtenu à partir de la 2-bromo-4,6-difluoroaniline selon le procédé décrit dans le stade A de l'Exemple 1.
*Point de fusion : 122-124 °C*

### Stade B : 2-bromo-6-cyclopropylamino-4-fluorobenzènesulfonamide

Le produit attendu est obtenu selon le procédé décrit dans le stade B de l'Exemple 1.

### Stade C : 8-bromo-4-cyclopropyl-6-fluoro-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 270-273 °C*

### Stade D : 8-bromo-4-cyclopropyl-6-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 197-199 °C*

### EXEMPLE 18 : 4-cyclopropyl-8-trifluorométhyl-3,4-dihydro-2H-1,2,4-benzothiadiazine 1,1-dioxyde

### Stade A : 2-fluoro-6-trifluorométhylbenzènesulfonamide

Le produit attendu est obtenu à partir de la 2-fluoro-6-trifluorométhylaniline selon le procédé décrit dans le stade A de l'Exemple 1.
*Point de fusion : 114-118 °C*

### Stade B : 2-cyclopropylamino-6-trifluorométhylbenzènesulfonamide

Le produit attendu est obtenu selon le procédé décrit dans le stade B de l'Exemple 1.

### Stade C : 4-cyclopropyl-8-trifluorométhyl-4H-1,2,4-benzothiadiazine 1,1-dioxyde

Le produit attendu est obtenu selon le procédé décrit (temps de chauffe de 10 heures à 130 °C) dans le stade C de l'Exemple 1.
*Point de fusion : 195-197 °C*

### Stade D : 4-cyclopropyl-8-trifluorométhyl-3,4-dihydro-2H-1,2,4-benzothiadiazine 1,1-dioxyde

Le produit attendu est obtenu selon le procédé décrit dans le stade D de l'Exemple 1.
*Point de fusion : 239-241 °C*

### EXEMPLE 19 : 5-chloro-4-cyclopropyl-3,4-dihydro-2H-1,2,4-benzothiadiazine 1,1-dioxyde

### Stade A : 3-chloro-2-fluorobenzènesulfonamide

Le produit attendu est obtenu à partir de la 3-chloro-2-fluoroaniline selon le procédé décrit dans le stade A de l'Exemple 1.
*Point de fusion : 149-153 °C*

### Stade B : 3-chloro-2-cyclopropylaminobenzènesulfonamide

Le produit attendu est obtenu selon le procédé décrit dans le stade B de l'Exemple 1.

### Stade C : 5-chloro-4-cyclopropyl-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 159-160 °C*

### Stade D : 5-chloro-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 142-143 °C*

### EXEMPLE 20 : 8-bromo-6-chloro-4-cyclopropyl-3,4-dihydro-2H-1,2,4-benzothiadiazine 1,1-dioxyde

### Stade A : 2-bromo-4-chloro-6-fluorobenzènesulfonamide

Le produit attendu est obtenu à partir de la 2-bromo-4-chloro-6-fluoroaniline selon le procédé décrit dans le stade A de l'Exemple 1.
*Point de fusion : 122-126 °C*

### Stade B : 2-bromo-4-chloro-6-cyclopropylaminobenzènesulfonamide

Le produit attendu est obtenu selon le procédé décrit dans le stade B de l'Exemple 1.

### Stade C : 8-bromo-6-chloro-4-cyclopropyl-4H-1,2,4-benzothiadiazine 1,1-dioxyde

Le produit attendu est obtenu selon le procédé décrit (temps de chauffe de 3 heures) dans le stade C de l'Exemple 1.

### Stade D : 8-bromo-6-chloro-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 177-180 °C*

### EXEMPLE 21 : 4-cyclopropyl-5-fluoro-3,4-dihydro-2H-1,2,4-benzothiadiazine 1,1-dioxyde

### StadeA : 2,3-difluorobenzènesulfonamide

Le produit attendu est obtenu à partir de la 2,3-difluoroaniline selon le procédé décrit dans le stade A de l'Exemple 1.
*Point de fusion : 151-153 °C*

### Stade B : 2-cyclopropylamino-3-fluorobenzènesulfonamide

Le produit attendu est obtenu selon le procédé décrit dans le stade B de l'Exemple 1.

### Stade C : 4-cyclopropyl-5-fluoro-4H-1,2,4-benzothiadiazine 1,1-dioxyde

Le produit attendu est obtenu selon le procédé décrit (temps de chauffe de 48 heures) dans le stade C de l'Exemple 1.
*Point de fusion : 151-152 °C*

### Stade D : 4-cyclopropyl-5-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 161-163 °C*

### EXEMPLE 22 : 6-bromo-4-cyclopropyl-3,4-dihydro-7-méthyl-2H-1,2,4-benzothiadiazine 1,1-dioxyde

### Stade A : 4-bromo-2-fluoro-5-méthylbenzènesulfonamide

Le produit attendu est obtenu à partir de la 4-bromo-2-fluoro-5-méthylaniline selon le procédé décrit dans le stade A de l'Exemple 1.
*Point de fusion : 124-125 °C*

### Stade B : 4-bromo-2-cyclopropylamino-5-méthylbenzènesulfonamide

Le produit attendu est obtenu selon le procédé décrit dans le stade B de l'Exemple 1.

### Stade C : 6-bromo-4-cyclopropyl-7-méthyl-4H-1,2,4-benzothiadiazine 1,1-dioxyde

Le produit attendu est obtenu selon le procédé décrit (temps de chauffe de 24 heures) dans le stade C de l'Exemple 1.
*Point de fusion : 219-220°C*

### Stade D : 6-bromo-4-cyclopropyl-3,4-dihydro-7-méthyl-2H-1,2,4-benzothiadiazine 1,1-dioxyde

Le produit attendu est obtenu selon le procédé décrit dans le stade D de l'Exemple 1.
*Point de fusion : 199-200 °C*

### EXEMPLE 23 : 5,6-dichloro-4-cyclopropyl-3,4-dihydro-2H-1,2,4-benzothiadiazine 1,1-dioxyde

### Stade A : 3,4-dichloro-2-fluorobenzènesulfonamide

Le produit attendu est obtenu à partir de la 3,4-dichloro-2-fluoroaniline selon le procédé décrit dans le stade A de l'Exemple 1.

### Stade B : 3,4-dichloro-2-cyclopropylaminobenzènesulfonamide

Le produit attendu est obtenu selon le procédé décrit dans le stade B de l'Exemple 1.

### Stade C : 5,6-dichloro-4-cyclopropyl-4H-1,2,4-benzothiadiazine 1,1-dioxyde

Le produit attendu est obtenu selon le procédé décrit (temps de chauffe de 2 heures à 130 °C) dans le stade C de l'Exemple 1.
*Point de fusion : 183-185 °C*

### Stade D : 5,6-dichloro-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 132-133 °C*

### EXEMPLE 24 : 8-bromo-4-cyclopropyl-3,4-dihydro-2H-1,2,4-benzothiadiazine 1,1-dioxyde

### Stade A : 2-bromo-6-fluorobenzènesulfonamide

Le produit attendu est obtenu à partir de la 2-bromo-6-fluoroaniline selon le procédé décrit dans le stade A de l'Exemple 1.
*Point de fusion : 185-187 °C*

### Stade B : 2-bromo-6-cyclopropylaminobenzènesulfonamide

Le produit attendu est obtenu selon le procédé décrit dans le stade B de l'Exemple 1.

### Stade C : 8-bromo-4-cyclopropyl-4H-1,2,4-benzothiadiazine 1,1-dioxyde

Le produit attendu est obtenu selon le procédé décrit (temps de chauffe de 1,5 heures) dans le stade C de l'Exemple 1.

### Stade D : 8-bromo-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 189-191 °C*

### EXEMPLE 25 : 4-cyclopropyl-5,7-difluoro-3,4-dihydro-2H-1,2,4-benzothiadiazine 1,1-dioxyde

### Stade A : 2,3,5-trifluorobenzènesulfonamide

Le produit attendu est obtenu à partir de la 2,3,5-trifluoroaniline selon le procédé décrit dans le stade A de l'Exemple 1.
*Point de fusion : 115-117 °C*

### Stade B : 2-cyclopropylamino-3,5-dfluorobenzènesumonamide

Le produit attendu est obtenu selon le procédé décrit (temps de chauffe de 40 heures) dans le stade B de l'Exemple 1.

### Stade C : 4-cyclopropyl-5,7-difluoro-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 160-163 °C*

### StadeD: 4-cyclopropyl-5,7-difluoro-3,4-dihydro-2H-1,2,4-benzothiadiazine 1,1-dioxyde

Le produit attendu est obtenu selon le procédé décrit dans le stade D de l'Exemple 1.
*Point de fusion : 149-151 °C*

### EXEMPLE 26 : 4-cyclopropyl-5-fluoro-3,4-dihydro-6-méthyl-2H-1,2,4-benzothiadiazine 1,1-dioxyde

### Stade A : 2,3-difluoro-4-méthylbenzènesulfonamide

Le produit attendu est obtenu à partir de la 2,3-difluoro-4-méthylaniline selon le procédé décrit dans le stade A de l'Exemple 1.
*Point de fusion : 171-173 °C*

### Stade B : 2-cyclopropylamino-3-fluoro-4-méthylbenzènesumonamide

Le produit attendu est obtenu selon le procédé décrit dans le stade B de l'Exemple 1.

### Stade C : 4-cyclopropyl-5-fluoro-6-méthyl-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 189-192 °C*

### Stade D : 4-cyclopropyl-5-fluoro-3,4-dihydro-6-méthyl-2H-1,2,4-benzothiadiazine 1,1-dioxyde

Le produit attendu est obtenu selon le procédé décrit dans le stade D de l'Exemple 1.
*Point de fusion : 135-138 °C*

### EXEMPLE 27 : 8-chloro-4-cyclopropyl-7-fluoro-3,4-dihydro-2H-1,2,4-benzothiadiazine 1,1-dioxyde

### Stade A : 2-chloro-3,6-difluorobenzènesulfonamide

Le produit attendu est obtenu à partir de la 2-chloro-3,6-difluoroaniline selon le procédé décrit dans le stade A de l'Exemple 1.
*Point de fusion : 175-178 °C*

### Stade B : 2-chloro-6-cyclopropylamino-3-fluorobenzènesulfonamide

Le produit attendu est obtenu selon le procédé décrit dans le stade B de l'Exemple 1.

### Stade C : 8-chloro-4-cyclopropyl-7-fluoro-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 260-263 °C*

### Stade D : 8-chloro-4-cyclopropyl-7-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 198-200 °C*

### EXEMPLE 28 : 4-cyclopropyl-3,4-dihydro-6-iodo-2H-1,2,4-benzothiadiazine 1,1-dioxyde

### Stade A : 2-fluoro-4-iodobenzènesulfonamide

Le produit attendu est obtenu à partir de la 2-fluoro-4-iodoaniline selon le procédé décrit dans le stade A de l'Exemple 1.

### Stade B : 2-cyclopropylamino-4-iodobenzènesulfonamide

Le produit attendu est obtenu selon le procédé décrit dans le stade B de l'Exemple 1.

### Stade C : 4-cyclopropyl-6-iodo-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 304-307 °C*

### Stade D : 4-cyclopropyl-3,4-dihydro-6-iodo-2H-1,2,4-benzothiadiazine 1,1-dioxyde

Le produit attendu est obtenu selon le procédé décrit dans le stade D de l'Exemple 1.
*Point de fusion : 183-185°C*

### EXEMPLE 29 : 4-cyclopropyl-8-fluoro-3,4-dihydro-2H-1,2,4-benzothiadiazine 1,1-dioxyde

### Stade A : 2-cyclopropylamino-6 fluorobenzènesulfonamide

Le produit attendu est obtenu à partir de la 2,6-difluorobenzènesulfonamide commerciale selon le procédé décrit dans le stade B de l'Exemple 1.

### Stade B : 4-cyclopropyl-8-fluoro-4H-1,2,4-benzothiadiazine 1,1-dioxyde

Le produit attendu est obtenu selon le procédé décrit (temps de chauffe de 24 heures) dans le stade C de l'Exemple 5.
*Point de fusion : 156-157 °C*

### Stade C : 4-cyclopropyl-8-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 177-179* °C

### EXEMPLE 30 : 6,8-dibromo-4-cyclopropyl-3,4-dihydro-2H-1,2,4-benzothiadiazine 1,1-dioxyde

### Stade A : 2,4-dibromo-6-fluorobenzènesulfonamide

Le produit attendu est obtenu à partir de la 2,4-dibromo-6-fluoroaniline selon le procédé décrit dans le stade A de l'Exemple 1.
*Point de fusion : 153-157 °C*

### Stade B : 2,4-dibromo-6-cyclopropylaminobenzènesulfonamide

Le produit attendu est obtenu selon le procédé décrit dans le stade B de l'Exemple 1.

### Stade C : 6,8-dibromo-4-cyclopropyl-4H-1,2,4-benzothiadiazine 1,1-dioxyde

Le produit attendu est obtenu selon le procédé décrit dans le stade C de l'Exemple 1.
*Point de fusion : 254-256 °C*

### Stade D : 6,8-dibromo-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 203-205 °C*

### EXEMPLE 31 : 5-chloro-4-cyclopropyl-6-fluoro-3,4-dihydro-2H-1,2,4-benzothiadiazine 1,1-dioxyde

### Stade A : 3-chloro-2,4-difluorobenzènesulfonamide

Le produit attendu est obtenu à partir de la 3-chloro-2,4-difluoroaniline selon le procédé décrit dans le stade A de l'Exemple 1.
*Point de fusion : 128-132 °C*

### Stade B : 3-chloro-2-cyclopropylamino-4-fluorobenzènesulfonamide

Le produit attendu est obtenu selon le procédé décrit dans le stade B de l'Exemple 1.

### Stade C : 5-chloro-4-cyclopropyl-6-fluoro-4H-1,2,4-benzothiadiazine 1,1-dioxyde

Le produit attendu est obtenu selon le procédé décrit (temps de chauffe de 2 heures à 130 °C) dans le stade C de l'Exemple 1.
*Point de fusion : 147-148 °C*

### Stade D : 5-chloro-4-cyclopropyl-6-fluoro-3,4-dihydro-2H-1,2,4-benzothiadiazine 1, 1-dioxyde

Le produit attendu est obtenu selon le procédé décrit dans le stade D de l'Exemple 1.
*Point de fusion : 141-143 °C*

### EXEMPLE 32 : 8-chloro-4-cyclopropyl-3,4-dihydro-2H-1,2,4-benzothiadiazine 1,1-dioxyde

### Stade A : 2-chloro-6-fluorobenzènesulfonamide

Le produit attendu est obtenu à partir de la 2-chloro-6-fluoroaniline selon le procédé décrit dans le stade A de l'Exemple 1.
*Point de fusion : 187-190 °C*

### Stade B : 2-chloro-6-cyclopropylaminobenzènesulfonamide

Le produit attendu est obtenu selon le procédé décrit dans le stade B de l'Exemple 1.

### Stade C : 8-chloro-4-cyclopropyl-4H-1,2,4-benzothiadiazine 1,1-dioxyde

Le produit attendu est obtenu selon le procédé décrit (temps de chauffe de 2 heures à 130 °C) dans le stade C de l'Exemple 1.
*Point de fusion : 207-209 °C*

### Stade D : 8-chloro-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 182-184 °C*

### EXEMPLE 33 : 6-bromo-4-cyclopropyl-8-trifluorométhyl-3,4-dihydro-2H-1,2,4-benzothiadiazine 1,1-dioxyde

### Stade A : 4-bromo-2-fluoro-6-trfluorométhylbenzènesulfonamide

Le produit attendu est obtenu à partir de la 4-bromo-2-fluoro-6-trifluorométhylaniline selon le procédé décrit dans le stade A de l'Exemple 1.
*Point de fusion : 133-135 °C*

### Stade B : 4-bromo-2-cyclopropylamino-6-trifluorométhylbenzènesulfonamide

Le produit attendu est obtenu selon le procédé décrit dans le stade B de l'Exemple 1.

### Stade C : 6-bromo-4-cyclopropyl-8-trifluorométhyl-4H-1,2,4-benzothiadiazine 1,1-dioxyde

Le produit attendu est obtenu selon le procédé décrit (temps de chauffe de 6 heures) dans le stade C de l'Exemple 1.
*Point de fusion : 253-254 °C*

### Stade D : 6-bromo-4-cyclopropyl-8-trifluorométhyl-3,4-dihydro-2H-1,2,4-benzothiadiazine 1, 1-dioxyde

Le produit attendu est obtenu selon le procédé décrit dans le stade D de l'Exemple 1.
*Point de fusion : 200-201 °C*

### EXEMPLE 34 : 6-bromo-4-cyclopropyl-7-fluoro-3,4-dihydro-2H-1,2,4-benzothiadiazine 1,1-dioxyde

### Stade A : 4-bromo-2,5-difluorobenzènesulfonamide

Le produit attendu est obtenu à partir de la 4-bromo-2,5-difluoroaniline selon le procédé décrit dans le stade A de l'Exemple 1.
*Point de fusion : 159-163 °C*

### Stade B : 4-bromo-2-cyclopropylamino-5-fluorobenzènesuifonamide

Le produit attendu est obtenu selon le procédé décrit dans le stade B de l'Exemple 1.

### Stade C : 6-bromo-4-cyclopropyl-7-fluoro-4H-1,2,4-benzothiadiazine 1,1-dioxyde

Le produit attendu est obtenu selon le procédé décrit (temps de chauffe de 24 heures) dans le stade C de l'Exemple 1.
*Point de fusion : 218-219 °C*

### Stade D : 6-bromo-4-cyclopropyl-7-fluoro-3,4-dihydro-2H-1,2,4-benzothiadiazine 1, 1-dioxyde

Le produit attendu est obtenu selon le procédé décrit dans le stade D de l'Exemple 1.
*Point de fusion : 163-165 °C*

### EXEMPLE 35 : 6-bromo-4-cyclopropyl-3,4-dihydro-2H-1,2,4-benzothiadiazine 1,1-dioxyde

### Stade A : 4-bromo-2-cyclopropylaminobenzènesulfonamide

Le produit attendu est obtenu à partir de la 4-bromo-2-fluorobenzènesulfonamide commerciale selon le procédé décrit dans le stade B de l'Exemple 1.

### Stade B : 6-bromo-4-cyclopropyl-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 268-271 °C*

### Stade C : 6-bromo-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 180-181* °C

### EXEMPLE 36 : 8-chloro-4-cyclopropyl-3,4-dihydro-5-méthyl-2H-1,2,4-benzothiadiazine 1,1-dioxyde

### Stade A : 6-chloro-2-fluoro-3-méthylbenzènesulfonamide

Le produit attendu est obtenu à partir de la 6-chloro-2-fluoro-3-méthylaniline selon le procédé décrit dans le stade A de l'Exemple 1.
*Point de fusion :* 174-176 *°C*

### Stade B : 6-chloro-2-cyclopropylamino-3-méthylbenzènesulfonamide

Le produit attendu est obtenu selon le procédé décrit (temps de chauffe de 48 heures) dans le stade B de l'Exemple 1.

### Stade C : 8-chloro-4-cyclopropyl-5-méthyl-4H-1,2,4-benzothiadiazine 1,1-dioxyde

Le produit attendu est obtenu selon le procédé décrit dans le stade C de l'Exemple 1.

### Stade D : 8-chloro-4-cyclopropyl-3,4-dihydro-5-méthyl-2H-1,2,4-benzothiadiazine 1,1-dioxyde

Le produit attendu est obtenu selon le procédé décrit dans le stade D de l'Exemple 1.
*Point de fusion : 215-217 °C*

### EXEMPLE 37 : 4-cyclopropyl-3,4-dihydro-6-méthyl-2H-1,2,4-benzothiadiazine 1,1-dioxyde

### Stade A : 2-fluoro-4-méthylbenzènesulfonamide

Le produit attendu est obtenu à partir de la 2-fluoro-4-méthylaniline selon le procédé décrit dans le stade A de l'Exemple 1.
*Point de fusion : 136-137 °C*

### Stade B : 2-cyclopropylamino-4-méthylbenzènesulfonamide

Le produit attendu est obtenu selon le procédé décrit (temps de chauffe de 24 heures) dans le stade B de l'Exemple 1.

### Stade C : 4-cyclopropyl-6-méthyl-4H-1,2,4-benzothiadiazine 1,1-dioxyde

Le produit attendu est obtenu selon le procédé décrit dans le stade C de l'Exemple 1.
*Point de fusion : 224-226 °C*

### Stade D : 4-cyclopropyl-3,4-dihydro-6-méthyl-2H-1,2,4-benzothiadiazine 1,1-dioxyde

Le produit attendu est obtenu selon le procédé décrit dans le stade D de l'Exemple 1.
*Point de fusion : 157-158 °C*

### EXEMPLE 38 : 4-cyclopropyl-6,8-difluoro-3,4-dihydro-2H-1,2,4-benzothiadiazine 1,1-dioxyde

### Stade A : 2,4,6-trifluorobenzènesulfonamide

Le produit attendu est obtenu à partir de la 2,4,6-trifluoroaniline selon le procédé décrit dans le stade A de l'Exemple 1.
*Point de fusion : 102-106 °C*

### Stade B : 2-cyclopropylamino-4,6-difluorobenzènesulfonamide

Le produit attendu est obtenu selon le procédé décrit dans le stade B de l'Exemple 1.

### Stade C : 4-cyclopropyl-6,8-difluoro-4H-1,2,4-benzothiadiazine 1,1-dioxyde

Le produit attendu est obtenu selon le procédé décrit (temps de chauffe de 24 heures) dans le stade C de l'Exemple 1.
*Point de fusion : 167-168 °C*

### Stade D : 4-cyclopropyl-6,8-difluoro-3,4-dihydro-2H-1,2,4-benzothiadiazine 1,1-dioxyde

Le produit attendu est obtenu selon le procédé décrit dans le stade D de l'Exemple 1.
*Point de fusion : 161-164 °C*

### EXEMPLE 39 : 4-cyclopropyl-5,6-difluoro-3,4-dihydro-2H-1,2,4-benzothiadiazine 1,1-dioxyde

### Stade A : 2,3,4-trifluorobenzènesulfonamide

Le produit attendu est obtenu à partir de la 2,3,4-trifluoroaniline selon le procédé décrit dans le stade A de l'Exemple 1.
*Point de fusion : 111-114 °C*

### Stade B : 2-cyclopropylamino-3,4-difluorobenzènesulfonamide

Le produit attendu est obtenu selon le procédé décrit dans le stade B de l'Exemple 1.

### Stade C : 4-cyclopropyl-5,6-difluoro-4H-1,2,4-benzothiadiazine 1,1-dioxyde

Le produit attendu est obtenu selon le procédé décrit (temps de chauffe de 6 heures) dans le stade C de l'Exemple 1.
*Point de fusion : 182-184 °C*

### Stade D : 4-cyclopropyl-5,6-difluoro-3,4-dihydro-2H-1,2,4-benzothiadiazine 1,1-dioxyde

Le produit attendu est obtenu selon le procédé décrit dans le stade D de l'Exemple 1.
*Point de fusion : 160-162 °C*

### EXEMPLE 40 : 7-bromo-4-cyclopropyl-3,4-dihydro-2H-1,2,4-benzothiadiazine 1,1-dioxyde

### Stade A : 5-bromo-2-fluorobenzènesulfonamide

Le produit attendu est obtenu à partir de la 5-bromo-2-fluoroaniline selon le procédé décrit dans le stade A de l'Exemple 1.
*Point de fusion : 149-151* °C

### Stade B : 5-bromo-2-cyclopropylaminobenzènesulfonamide

Le produit attendu est obtenu selon le procédé décrit dans le stade B de l'Exemple 1.

### Stade C : 7-bromo-4-cyclopropyl-4H-1,2,4-benzothiadiazine 1,1-dioxyde

Le produit attendu est obtenu selon le procédé décrit (temps de chauffe de 5 heures à 130 °C) dans le stade C de l'Exemple 1.
*Point de fusion : 242-244 °C*

### Stade D : 7-bromo-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 178-179 °C*

### EXEMPLE 41 : 4-cyclopropyl-3,4-dihydro-7-nitro-2H-1,2,4-benzothiadiazine 1,1-dioxyde

### Stade A : 2-chloro-5-nitrobenzènesulfonamide

Une portion d'acide acétique glacial (160 mL) est saturé pendant 30 minutes par de l'anhydride sulfureux gazeux. A cette solution refroidie sur bain de glace est ajoutée sous agitation une solution aqueuse de CuCl₂ (7 g dans 20 mL) (suspension A).

La 2-chloro-5-nitroaniline (15 g) est dissoute dans un mélange d'acide acétique glacial (160 mL) et de HCl concentré (40 mL). A cette solution refroidie sur bain de glace et de sel (-5 °C) est ajoutée goutte à goutte et sous agitation une solution aqueuse de NaNO₂ (8 g dans 20 mL). Au terme de l'addition, cette solution est mélangée lentement sous agitation à la suspension A. Après 15 minutes d'agitation, la suspension est versée sur de la glace (400 g). Le précipité formé est recueilli par filtration, lavé à l'eau et redissous immédiatement dans le dioxanne (150 mL). La solution obtenue est ajoutée progressivement sous agitation à une solution aqueuse concentrée d'ammoniaque (300 mL) préalablement refroidie sur bain de glace. Après 30 minutes d'agitation, le solvant organique et une partie de l'ammoniaque sont éliminés par évaporation sous dépression. La solution/suspension aqueuse obtenue est ajustée à pH neutre par addition de HCl 6 N. Le précipité formé est recueilli par filtration et lavé à l'eau. Il est mis en suspension dans de l'eau (200 mL) et additionné de NaOH 10 % jusqu'à pH clairement alcalin. La suspension est chauffée pour favoriser la dissolution du produit du titre. L'insoluble persistant est éliminé par filtration à chaud. Le filtrat refroidi est ajusté à pH neutre ou légèrement acide par addition de HCl 6 N. Le précipité est recueilli sur filtre, lavé à l'eau et séché.
*Point de fusion : 180-183 °C*

### Stade B : 2-cyclopropylamino-5-nitrobenzènesulfonamide

Dans une enceinte hermétique contenant un mélange de dioxanne (70 mL) et de cyclopropylamine (3,5 mL) est introduit le 2-chloro-5-nitrobenzènesulfonamide (5 g) préparé lors de l'étape précédente. L'enceinte hermétique est placée à l'étuve à 100 °C pendant 24 heures. Après ce laps de temps, le solvant et le réactif sont éliminés par concentration sous dépression. Le résidu est repris par du méthanol (20 mL) et l'insoluble constitué du produit du titre est recueilli par filtration, lavé au méthanol et séché.

### Stade C : 4-cyclopropyl-7-nitro-4H-1,2,4-benzothiadiazine 1,1-dioxyde

Dans un ballon, le mélange de 2-cyclopropylamino-5-nitrobenzènesulfonamide (5 g), issue de l'étape précédente, et d'orthoformiate d'éthyle (50 mL) est chauffé en vase ouvert à 130 °C pendant 3 heures. La suspension obtenue est refroidie sur bain de glace et l'insoluble est recueilli par filtration, lavé à l'éther et séché.
*Point de fusion : 233-235 °C*

### Stade D : 4-cyclopropyl-3,4-dihydro-7-nitro-2H-1,2,4-benzothiadiazine 1,1-dioxyde

Le produit attendu est obtenu selon le procédé décrit dans le stade D de l'Exemple 1.
*Point de fusion : 198-201 °C*

### EXEMPLE 42 : 7-acétamido-4-cyclopropyl-3,4-dihydro-2H-1,2,4-benzothiadiazine 1,1-dioxyde

### Stade A : 7-amino-4-cyclopropyl-4H-1,2,4-benzothiadiazine 1,1-dioxyde

Une solution de 4-cyclopropyl-7-nitro-4*H*-1,2,4-benzothiadiazine 1,1-dioxyde (5 g ; préparé lors du stade C de l'Exemple 41) dans l'éthanol (180 mL) est additionné de charbon palladié à 10 % (500 mg). La suspension est placée à l'hydrogénateur sous 10 atmosphères de H₂ pendant 30 minutes à température ambiante. La suspension est concentrée à siccité sous dépression et le résidu est repris par de l'acétone bouillant (300 mL). L'insoluble est éliminé par filtration à chaud et lavé avec de l'acétone bouillant. Le filtrat est concentré à siccité et le résidu est recristallisé dans le méthanol.
*Point de fusion : 283-285 °C*

### Stade B : 7-acétamido-4-cyclopropyl-3,4-dihydro-2H-1,2,4-benzothiadiazine 1,1-dioxyde

La solution de 7-amino-4-cyclopropyl-4*H*-1,2,4-benzothiadiazine 1,1-dioxyde préparé dans l'étape précédente (0,5 g) dans le dioxanne (20 mL) est additionnée de chlorure d'acétyle (0,5 mL) et mise sous agitation à température ambiante pendant 16 heures. Après élimination du solvant par distillation sous pression réduite, le résidu est repris par de l'eau et le précipité de 7-acétamido-4-cyclopropyl-4*H*-1,2,4-benzothiadiazine 1,1-dioxyde est recueilli par filtration, lavé à l'eau et séché. Il est engagé directement dans l'étape suivante afin d'obtenir le produit attendu selon le procédé décrit dans le stade D de l'Exemple 1.
*Point de fusion : 230-232 °C*

### EXEMPLE 43 : 6,7-dichloro-4-cyclobutyl-3,4-dihydro-2H-1,2,4-benzothiadiazine 1,1-dioxyde

### Stade A : 4,5-dichloro-2-cyc/obutylaminobenzènesulfonamide

Le produit attendu est obtenu à partir de la 4,5-dichloro-2-fluoroaniline selon le procédé décrit dans les stades A et B de l'Exemple 1 avec l'addition de la cyclobutylamine lors de l'étape B.

### Stade B : 6,7-dichloro-4-cyclobutyl-4H-1,2,4-benzothiadiazine 1,1-dioxyde

Le produit attendu est obtenu selon le procédé décrit (temps de chauffe de 24 heures) dans le stade C de l'Exemple 1.
*Point de fusion : 238-240 °C*

### Stade C : 6,7-dichloro-4-cyclobutyl-3,4-dihydro-2H-1,2,4-benzothiadiazine 1,1-dioxyde

Le produit attendu est obtenu selon le procédé décrit dans le stade D de l'Exemple 1.
*Point de fusion : 161-163 °C*

### EXEMPLE 44 : 7-chloro-4-cyclobutyl-3,4-dihydro-2H-1,2,4-benzothiadiazine 1,1-dioxyde

### Stade A : 4-cyclobutyl-7-nitro-4H-1,2,4-benzothiadiazine 1,1-dioxyde

Le produit attendu est obtenu à partir de la 2-chloro-5-nitroaniline selon le procédé décrit dans les stades A, B et C de l'Exemple 41 avec l'addition de la cyclobutylamine lors de l'étape B.
*Point de fusion : 229-232 °C*

### Stade B : 7-amino-4-cyclobutyl-4H-1,2,4-benzothiadiazine 1,1-dioxyde

Le produit attendu est obtenu selon le procédé décrit dans le stade A de l'Exemple 42.
*Point de fusion : 278-280 °C*

### Stade C : 7-chloro-4-cyclobutyl-4H-1,2,4-benzothiadiazine 1,1-dioxyde

Une solution de CuSO₄.5H₂O (84 g) et de NaCl (22,5 g) dans l'eau (200 mL) est refroidie sur bain de glace et additionnée goutte à goutte d'une solution aqueuse de Na₂S₂O₅ (22,5 g dans 100 mL). Après 15 minutes d'agitation, le précipité de Cu₂Cl₂ est recueilli par filtration et lavé à l'eau.

Une solution de 7-amino-4-cyclobutyl-4*H*-1,2,4-benzothiadiazine 1,1-dioxyde préparé lors de l'étape précédente (3,48 g) dans le HCl 6 N (40 mL) est refroidie sur bain de glace, puis additionnée goutte à goutte d'une solution aqueuse de NaNO₂ (2 g dans 15 mL). La solution obtenue est ajoutée progressivement à la solution de Cu₂Cl₂ dans le HCl concentré (30 mL). Après 30 minutes d'agitation à température ambiante, le milieu réactionnel est additionné d'eau (150 mL) et le précipité obtenu est recueilli par filtration, lavé à l'eau et séché.
*Point de fusion : 229-231 °C*

### Stade D : 7-chloro-4-cyclobutyl-3,4-dihydro-2H-1,2,4-benzothiadiazine 1,1-dioxyde

La solution de 7-chloro-4-cyclobutyl-4*H*-1,2,4-benzothiadiazine 1,1-dioxyde issue de l'étape précédente (3 g) dans l'isopropanol (100 mL) est additionnée de NaBH₄ (2,5 g) finement broyé, puis chauffée pendant 10 minutes à 55 °C. Le solvant est éliminé par évaporation sous dépression. Le résidu est repris par de l'eau (100 mL) et amené à pH acide par addition de HCl 6 N. Le produit du titre est extrait par le chloroforme (3 x 50 mL). La phase organique est séchée sur MgSO₄ et filtrée. Le filtrat est évaporé à siccité et le résidu est purifié par chromatographie sur colonne de silice (phase mobile : chloroforme). Le produit obtenu est recristallisé dans le méthanol.
*Point de fusion : 162-164 °C*

### EXEMPLE 45 : 6-chloro-4-cyclobutyl-3,4-dihydro-2H-1,2,4-benzothiadiazine 1,1-dioxyde

### Stade A : 4-chloro-2-cyclobutylaminobenzènesulfonamide

Le produit attendu est obtenu à partir de la 4-chloro-2-fluoroaniline selon le procédé décrit dans les stades A et B de l'Exemple 1 avec l'addition de la cyclobutylamine lors de l'étape B.

### Stade B : 6-chloro-4-cyclobutyl-4H-1,2,4-benzothiadiazine 1,1-dioxyde

Le produit attendu est obtenu selon le procédé décrit (temps de chauffe de 6 heures à 130 °C) dans le stade C de l'Exemple 1.
*Point de fusion : 224-226 °C*

### Stade C : 6-chloro-4-cyclobutyl-3,4-dihydro-2H-1,2,4-benzothiadiazine 1,1-dioxyde

Le produit attendu est obtenu selon le procédé décrit dans le stade D de l'Exemple 1.
*Point de fusion : 183-185 °C*

### EXEMPLE 46 : 4-cyclobutyl-3,4-dihydro-7-nitro-2H-1,2,4-benzothiadiazine 1,1-dioxyde

Le produit attendu est obtenu à partir de la 4-cyclobutyl-7-nitro-4*H*-1,2,4-benzothiadiazine 1,1-dioxyde (préparée lors du stade A de l'Exemple 44) selon le procédé décrit dans le stade D de l'Exemple 1.
*Point de fusion : 188-190 °C*

### EXEMPLE 47 : 6,7-dichloro-4-cyclopentyl-3,4-dihydro-2H-1,2,4-benzothiadiazine 1,1-dioxyde

### Stade A : 4,5-dichloro-2-cyclopentylaminobenzènesulfonamide

Le produit attendu est obtenu à partir de la 4,5-dichloro-2-fluoroaniline selon le procédé décrit dans les stades A et B de l'Exemple 1 avec l'addition de la cyclopentylamine lors de l'étape B.

### Stade B : 6,7-dichloro-4-cyclopentyl-4H-1,2,4-benzothiadiazine 1, 1-dioxyde

Le produit attendu est obtenu selon le procédé décrit (temps de chauffe de 72 heures à 140 °C) dans le stade C de l'Exemple 5.
*Point de fusion : 189-191 °C*

### Stade C : 6,7-dichloro-4-cyclopentyl-3,4-dihydro-2H-1,2,4-benzothiadiazine 1,1-dioxyde

Le produit attendu est obtenu selon le procédé décrit dans le stade D de l'Exemple 1.
*Point de fusion : 221-224 °C*

### EXEMPLE 48 : 7-chloro-4-cyclopentyl-3,4-dihydro-2H-1,2,4-benzothiadiazine 1,1-dioxyde

### Stade A : 5-chloro-2-cyclopentylaminobenzènesulfonamide

Le produit attendu est obtenu à partir de la 5-chloro-2-fluoroaniline selon le procédé décrit dans les stades A et B de l'Exemple 1 avec l'addition de la cyclopentylamine lors de l'étape B.

### Stade B : 7-chloro-4-cyclopentyl-4H-1,2,4-benzothiadiazine 1,1-dioxyde

Le produit attendu est obtenu selon le procédé décrit (temps de chauffe de 96 heures) dans le stade C de l'Exemple 5.
*Point de fusion : 218-220 °C*

### Stade C : 7-chloro-4-cyclopentyl-3,4-dihydro-2H-1,2,4-benzothiadiazine 1,1-dioxyde

Le produit attendu est obtenu selon le procédé décrit dans le stade D de l'Exemple 1.
*Point de fusion : 193-194 °C*

### EXEMPLE 49 : 6-chloro-4-cyclopentyl-3,4-dihydro-2H-1,2,4-benzothiadiazine 1,1-dioxyde

### Stade A : 4-chloro-2-cyclopentylaminobenzènesulfonamide

Le produit attendu est obtenu à partir de la 4-chloro-2-fluoroaniline selon le procédé décrit dans les stades A et B de l'Exemple 1 avec l'addition de la cyclopentylamine lors de l'étape B.

### Stade B : 6-chloro-4-cyclopentyl-4H-1,2,4-benzothiadiazine 1,1-dioxyde

Le produit attendu est obtenu selon le procédé décrit dans le stade C de l'Exemple 5.
*Point de fusion : 170-172 °C*

### Stade C : 6-chloro-4-cyclopentyl-3,4-dihydro-2H-1,2,4-benzothiadiazine 1,1-dioxyde

Le produit attendu est obtenu selon le procédé décrit dans le stade D de l'Exemple 1.
*Point de fusion : 175-177 °C*

### EXEMPLE 50 : 6,7-dichloro-4-cyclohexyl-3,4-dihydro-2H-1,2,4-benzothiadiazine 1,1-dioxyde

### Stade A : 4,5-dichloro-2-cyclohexylaminobenzènesulfonamide

Le produit attendu est obtenu à partir de la 4,5-dichloro-2-fluoroaniline selon le procédé décrit dans les stades A et B de l'Exemple 1 avec l'addition de la cyclohexylamine chauffée à 80 °C en enceinte hermétique pendant 72 heures lors de l'étape B.

### Stade B : 6,7-dichloro-4-cyclohexyl-4H-1,2,4-benzothiadiazine 1,1-dioxyde

Le produit attendu est obtenu selon le procédé décrit (temps de chauffe de 72 heures à 140 °C) dans le stade C de l'Exemple 5.
*Point de fusion : 219-222 °C*

### Stade C : 6,7-dichloro-4-cyclohexyl-3,4-dihydro-2H-1,2,4-benzothiadiazine 1,1-dioxyde

Le produit attendu est obtenu selon le procédé décrit dans le stade D de l'Exemple 1.
*Point de fusion : 207-209 °C*

### EXEMPLE 51 : 7-chloro-4-cyclohexyl-3,4-dihydro-2H-1,2,4-benzothiadiazine 1,1-dioxyde

### Stade A : 5-chloro-2-cyclohexylaminobenzènesulfonamide

Le produit attendu est obtenu à partir de la 5-chloro-2-fluoroaniline selon le procédé décrit dans les stades A et B de l'Exemple 1 avec l'addition de la cyclohexylamine lors de l'étape B.

### Stade B : 7-chloro-4-cyclohexyl-4H-1,2,4-benzothiadiazine 1,1-dioxyde

Le produit attendu est obtenu selon le procédé décrit (temps de chauffe de 5 heures à 130 °C) dans le stade C de l'Exemple 1.
*Point de fusion : 209-211 °C*

### Stade C : 7-chloro-4-cyclohexyl-3,4-dihydro-2H-1,2,4-benzothiadiazine 1,1-dioxyde

Le produit attendu est obtenu selon le procédé décrit dans le stade D de l'Exemple 1.
*Point de fusion : 233-235 °C*

### EXEMPLE 52 : 6-chloro-4-cyclohexyl-3,4-dihydro-2H-1,2,4-benzothiadiazine 1,1-dioxyde

### Stade A : 4-chloro-2-cyclohexylaminobenzènesulfonamide

Le produit attendu est obtenu à partir de la 4-chloro-2-fluoroaniline selon le procédé décrit dans les stades A et B de l'Exemple 1 avec l'addition de la cyclohexylamine lors de l'étape B.

### Stade B : 6-chloro-4-cyclohexyl-4H-1,2,4-benzothiadiazine 1,1-dioxyde

Le produit attendu est obtenu selon le procédé décrit (temps de chauffe de 48 heures à 130 °C) dans le stade C de l'Exemple 1.
*Point de fusion : 189-191 °C*

### Stade C : 6-chloro-4-cyclohexyl-3,4-dihydro-2H-1,2,4-benzothiadiazine 1,1-dioxyde

Le produit attendu est obtenu selon le procédé décrit dans le stade D de l'Exemple 1.
*Point de fusion : 178-180 °C*

### EXEMPLE 53 : 6,7-dichloro-4-cycloheptyl-3,4-dihydro-2H-1,2,4-benzothiadiazine 1,1-dioxyde

### Stade A : 4,5-dichloro-2-cycloheptylaminobenzènesulfonamide

Le produit attendu est obtenu à partir de la 4,5-dichloro-2-fluoroaniline selon le procédé décrit dans les stades A et B de l'Exemple 1 avec l'addition de la cycloheptylamine lors de l'étape B.

### Stade B : 6,7-dichloro-4-cycloheptyl-4H-1,2,4-benzothiadiazine 1,1-dioxyde

Le produit attendu est obtenu selon le procédé décrit (temps de chauffe de 72 heures à 140 °C) dans le stade C de l'Exemple 5.
*Point de fusion : 222-224 °C*

### Stade C : 6,7-dichloro-4-cycloheptyl-3,4-dihydro-2H-1,2,4-benzothiadiazine 1,1-dioxyde

Le produit attendu est obtenu selon le procédé décrit dans le stade D de l'Exemple 1.
*Point de fusion : 191-192 **°C***

### EXEMPLE 54 : 7-chloro-4-cycloheptyl-3,4-dihydro-2H-1,2,4-benzothiadiazine 1,1-dioxyde

### Stade A : 5-chloro-2-cycloheptylaminobenzènesulfonamide

Le produit attendu est obtenu à partir de la 5-chloro-2-fluoroaniline selon le procédé décrit dans les stades A et B de l'Exemple 1 avec l'addition de la cycloheptylamine lors de l'étape B.

### Stade B : 7-chloro-4-cycloheptyl-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 241-243 °C*

### Stade C : 7-chloro-4-cycloheptyl-3,4-dihydro-2H-1,2,4-benzothiadiazine 1,1-dioxyde

Le produit attendu est obtenu selon le procédé décrit dans le stade D de l'Exemple 1.
*Point de fusion : 216-218 °C*

### EXEMPLE 55 : 6-chloro-4-cycloheptyl-3,4-dihydro-2H-1,2,4-benzothiadiazine 1,1-dioxyde

### Stade A : 4-chloro-2-cycloheptylaminobenzènesulfonamide

Le produit attendu est obtenu à partir de la 4-chloro-2-fluoroaniline selon le procédé décrit dans les stades A et B de l'Exemple 1 avec l'addition de la cycloheptylamine lors de l'étape B.

### Stade B : 6-chloro-4-cycloheptyl-4H-1,2,4-benzothiadiazine 1,1-dioxyde

Le produit attendu est obtenu selon le procédé décrit (temps de chauffe de 72 heures à 130 °C) dans le stade C de l'Exemple 1.
*Point de fusion : 209-211 °C*

### Stade C : 6-chloro-4-cycloheptyl-3,4-dihydro-2H-1,2,4-benzothiadiazine 1,1-dioxyde

Le produit attendu est obtenu selon le procédé décrit dans le stade D de l'Exemple 1.
*Point de fusion : 149-151 °C*

### EXEMPLE 56 : 6,7-dichloro-4-cyclooctyl-3,4-dihydro-2H-1,2,4-benzothiadiazine 1,1-dioxyde

### Stade A : 4,5-dichloro-2-cyclooctylaminobenzènesulfonamide

Le produit attendu est obtenu à partir de la 4,5-dichloro-2-fluoroaniline selon le procédé décrit dans les stades A et B de l'Exemple 1 avec l'addition de la cyclooctylamine chauffée à 80 °C en enceinte hermétique pendant 72 heures lors de l'étape B.

### Stade B : 6,7-dichloro-4-cyclooctyl-4H-1,2,4-benzothiadiazine 1,1-dioxyde

Le produit attendu est obtenu selon le procédé décrit (temps de chauffe de 72 heures à 140 °C) dans le stade C de l'Exemple 5.
*Point de fusion : 229-231 °C*

### Stade C : 6,7-dichloro-4-cyclooctyl-3,4-dihydro-2H-1,2,4-benzothiadiazine 1,1-dioxyde

Le produit attendu est obtenu selon le procédé décrit dans le stade D de l'Exemple 1.
*Point de fusion : 154-155 °C*

### EXEMPLE 57 : 7-chloro-4-cyclooctyl-3,4-dihydro-2H-1,2,4-benzothiadiazine 1,1-dioxyde

### Stade A : 5-chloro-2-cyclooctylaminobenzènesulfonamide

Le produit attendu est obtenu à partir de la 5-chloro-2-fluoroaniline selon le procédé décrit dans les stades A et B de l'Exemple 1 avec l'addition de la cyclooctylamine lors de l'étape B.

### Stade B : 7-chloro-4-cyclooctyl-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 235-23 7 °C*

### Stade C : 7-chloro-4-cyclooctyl-3,4-dihydro-2H-1,2,4-benzothiadiazine 1,1-dioxyde

Le produit attendu est obtenu selon le procédé décrit dans le stade D de l'Exemple 1.
*Point de fusion : 184-186 °C*

### EXEMPLE 58 : 6-chloro-4-cyclooctyl-3,4-dihydro-2H-1,2,4-benzothiadiazine 1,1-dioxyde

### Stade A : 4-chloro-2-cyclooctylaminobenzènesulfonamide

Le produit attendu est obtenu à partir de la 4-chloro-2-fluoroaniline selon le procédé décrit dans les stades A et B de l'Exemple 1 avec l'addition de la cyclooctylamine lors de l'étape B.

### Stade B : 6-chloro-4-cyclooctyl-4H-1,2,4-benzothiadiazine 1,1-dioxyde

Le produit attendu est obtenu selon le procédé décrit (temps de chauffe de 48 heures à 130 °C) dans le stade C de l'Exemple 1.
*Point de fusion : 209-211 °C*

### Stade C : 6-chloro-4-cyclooctyl-3,4-dihydro-2H-1,2,4-benzothiadiazine 1,1-dioxyde

Le produit attendu est obtenu selon le procédé décrit dans le stade D de l'Exemple 1.
*Point de fusion : 141-143 °C*

### EXEMPLE 59 : 6,7-dichloro-4-(1-méthyl)cyclopropyl-3,4-dihydro-2H-1,2,4-benzothiadiazine 1,1-dioxyde

### Stade A : 4,5-dichloro-2-(1-méthyl)cyclopropylaminobenzènesulfonamide

Le produit attendu est obtenu à partir de la 4,5-dichloro-2-fluoroaniline selon le procédé décrit dans les stades A et B de l'Exemple 1 avec l'addition de la (1-méthyl)cyclopropylamine lors de l'étape B.
*Point de fusion : 130-132 °C*

### Stade B : 6,7-dichloro-4-(1-méthyl)cyclopropyl-4H-1,2,4-benzothiadiazine 1,1-dioxyde

Le produit attendu est obtenu selon le procédé décrit (temps de chauffe de 24 heures) dans le stade C de l'Exemple 1.
*Point de fusion : 230-232 °C*

### Stade C : 6,7-dichloro-4-(1-méthyl)cyclopropyl-2H-1,2,4-benzothiadiazine 1,1-dioxyde

Le produit attendu est obtenu selon le procédé décrit dans le stade D de l'Exemple 1.
*Point de fusion : 162-163 °C*

### EXEMPLE 60 : 7-chloro-4-cydopropylméthyl-3,4-dihydro-2H-1,2,4-benzothiadiazine 1,1-dioxyde

### Stade A : 7-chloro-4H-1,2,4-benzothiadiazine 1,1-dioxyde

Le mélange de 2-amino-5-chlorobenzènesulfonamide (obtenu selon J. Chem. Soc. Perkin I, 1043-1047, 1979) et d'orthoformiate d'éthyle (40 mL) est porté à ébullition pendant 30 minutes en vase ouvert. Le volume du milieu est réduit de moitié sous pression réduite. Le précipité obtenu est recueilli par filtration, lavé à l'éther et séché.
*Point de fusion : 243-244 °C*

### Stade B : 7-chloro-4-cyclopropylméthyl-4H-1,2,4-benzothiadiazine 1,1-dioxyde

La solution de 7-chloro-4*H*-1,2,4-benzothiadiazine 1,1-dioxyde (1 g) dans l'acétonitrile (40 mL) est additionnée de carbonate potassique (2,5 g) et de bromure de cyclopropylméthyle (0,7 mL) et le mélange est porté à reflux pendant 24 heures. Le milieu réactionnel est évaporé sous pression réduite et le résidu est repris par de l'eau (40 mL). L'insoluble est recueilli par filtration, lavé à l'eau et séché. Il est recristallisé dans le méthanol.
*Point de fusion : 183-185 °C*

### Stade C : 7-chloro-4-cyclopropylméthyl-3,4-dihydro-2H-1,2,4-benzothiadiazine 1,1-dioxyde

La solution de 7-chloro-4-cyclopropylméthyl-4*H*-1,2,4-benzothiadiazine 1,1-dioxyde (0,7 g) dans l'isopropanol (50 mL) est additionnée de borohydrure sodique (0,2 g) et le mélange est chauffé à 50 °C pendant 5 minutes. Le milieu réactionnel est évaporé sous pression réduite et le résidu est repris par de l'eau (30 mL). Le pH du milieu est ajusté à 5-6 par ajout d'acide chlorhydrique 6 N et la suspension est extraite trois fois par le chloroforme (30 mL). Les phases organiques sont rassemblées, séchées sur MgSO₄ anhydre et filtrées. Le filtrat est évaporé sous pression réduite et le résidu est repris par du méthanol (5 mL). A la solution méthanolique est ajoutée de l'eau (50 mL) et le précipité qui apparaît est recueilli par filtration, lavé à l'eau et séché.
*Point de fusion : 106-109 °C*

### EXEMPLE 61 : 4-cyclopropylméthyl-7-fluoro-3,4-dihydro-2H-1,2,4-benzothiadiazine 1,1-dioxyde

### Stade A : 7-fluoro-4H-1,2,4-benzothiadiazine 1,1-dioxyde

Le produit attendu est obtenu à partir de la 2-amino-5-fluorobenzènesulfonamide selon le procédé décrit dans le stade A de l'Exemple 60.
*Point de fusion : 269-270 °C*

### Stade B : 4-cyclopropylméthyl-7-fluoro-4H-1,2,4-benzothiadiazine 1,1-dioxyde

Le produit attendu est obtenu selon le procédé décrit dans le stade B de l'Exemple 60.
*Point de fusion : 155-157 °C*

### Stade C : 4-cyclopropylméthyl-7-fluoro-3,4-dihydro-2H-1,2,4-benzothiadiazine 1,1-dioxyde

Le produit attendu est obtenu selon le procédé décrit dans le stade C de l'Exemple 60.
*Point de fusion : 102-105 °C*

### EXEMPLE 62 : 4-cyclopropyl-7,8-difluoro-3,4-dihydro-2H-1,2,4-benzothiadiazine 1,1-dioxyde

### Stade A : 2,3,6-trifluorobenzènesulfonamide

Le produit attendu est obtenu à partir de la 2,3,6-trifluoroaniline selon le procédé décrit dans le stade A de l'Exemple 1.
*Point de fusion : 148-151 °C*

### Stade B : 2-cyclopropylamino-5,6-difluorobenzènesulfonamide

Le produit attendu est obtenu selon le procédé décrit dans le stade B de l'Exemple 1.

### Stade C : 4-cyclopropyl-7,8-difluoro-4H-1,2,4-benzothiadiazine 1,1-dioxyde

Le produit attendu est obtenu selon le procédé décrit (temps de chauffe de 2 heures à 130 °C) dans le stade C de l'Exemple 1.
*Point de fusion : 205-206 °C*

### Stade D : 4-cyclopropyl-7,8-difluoro-3,4-dihydro-2H-1,2,4-benzothiadiazine 1,1-dioxyde

Le produit attendu est obtenu selon le procédé décrit dans le stade D de l'Exemple 1.
*Point de fusion : 199-201 °C*

### EXEMPLE 63 : 6-cyano-4-cyclopropyl-7-fluoro-3,4-dihydro-2H-1,2,4-benzothiadiazine 1,1-dioxyde

### Stade A : 2,5-difluoro-4-sulfamoylbenzonitrile

Le produit attendu est obtenu à partir de la 4-cyano-2,5-difluoroaniline selon le procédé décrit dans le stade A de l'Exemple 1.

### Stade B : 2-cyclopropylamino-2-fluoro-4-sulfamoylbenzonitrile

Le produit attendu est obtenu selon le procédé décrit dans le stade B de l'Exemple 1.

### Stade C : 6-cyano-4-cyclopropyl-7-fluoro-4H-1,2,4-benzothiadiazine 1,1-dioxyde

Le produit attendu est obtenu selon le procédé décrit (temps de chauffe de 24 heures à 130 °C) dans le stade C de l'Exemple 1.
*Point de fusion : 205-207 °C*

### Stade D : 6-cyano-4-cyclopropyl-7-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 208-210 °C*

### EXEMPLE 64 : 6-bromo-4-cyclopropyl-8-fluoro-3,4-dihydro-2H-1,2,4-benzothiadiazine 1,1-dioxyde

### Stade A : 4-bromo-2,6-difluorobenzènesulfonamide

Le produit attendu est obtenu à partir de la 4-bromo-2,6-difluoroaniline selon le procédé décrit dans le stade A de l'Exemple 1.
*Point de fusion : 142-146 °C*

### Stade B : 4-bromo-2-cyclopropylamino-6-fluorobenzènesulfonamide

Le produit attendu est obtenu selon le procédé décrit dans le stade B de l'Exemple 1.

### Stade C : 6-bromo-4-cyclopropyl-8-fluoro-4H-1,2,4-benzothiadiazine 1,1-dioxyde

Le produit attendu est obtenu selon le procédé décrit (temps de chauffe de 72 heures à 130 °C) dans le stade C de l'Exemple 1.
*Point de fusion : 285-287 °C*

### Stade D : 6-bromo-4-cyclopropyl-8-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 195-198 °C*

### EXEMPLE 65 : 6-bromo-4-cyclopropyl-5-fluoro-3,4-dihydro-2H-1,2,4-benzothiadiazine 1,1-dioxyde

### Stade A : 4-bromo-2,3-difluorobenzènesulfonamide

Le produit attendu est obtenu à partir de la 4-bromo-2,3-difluoroaniline selon le procédé décrit dans le stade A de l'Exemple 1.
*Point de fusion : 155-15 7 °C*

### Stade B : 4-bromo-2-cyc/opropylamino-3-fluorobenzènesulfonamide

Le produit attendu est obtenu selon le procédé décrit dans le stade B de l'Exemple 1.

### Stade C: 6-bromo-4-cyclopropyl-5-fluoro-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 244-247 °C*

### Stade D : 6-bromo-4-cyclopropyl-5-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 197-199 °C*

### EXEMPLE 66 : 7-bromo-4-cyclopropyl-6-fluoro-3,4-dihydro-2H-1,2,4-benzothiadiazine 1,1-dioxyde

### Stade A : 5-bromo-2,4-difluorobenzènesulfonamide

Le produit attendu est obtenu à partir de la 5-bromo-2,4-difluoroaniline selon le procédé décrit dans le stade A de l'Exemple 1.
*Point de fusion : 109-111 °C*

### Stade B : 5-bromo-2-cyc/opropylamino-4-fluorobenzènesulfonamide

Le produit attendu est obtenu selon le procédé décrit dans le stade B de l'Exemple 1.

### Stade C : 7-bromo-4-cyclopropyl-6-fluoro-4H-1,2,4-benzothiadiazine 1,1-dioxyde

Le produit attendu est obtenu selon le procédé décrit (temps de chauffe de 4 heures à 130 °C) dans le stade C de l'Exemple 1.
*Point de fusion : 228-231 °C*

### Stade D : 7-bromo-4-cyclopropyl-6-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 175-177 °C*

### EXEMPLE 67 : 4-cyclopropyl-5,7,8-trifluoro-3,4-dihydro-2H-1,2,4-benzothiadiazine 1,1-dioxyde

### Stade A : 2-cyclopropylamino-3,5,6-trifluorobenzènesulfonamide

Le produit attendu est obtenu à partir de la 2,3,5,6-tétrafluorobenzènesulfonamide commerciale selon le procédé décrit dans le stade B de l'Exemple 1.

### Stade B : 4-cyclopropyl-5,7,8-trifluoro-4H-1,2,4-benzothiadiazine 1,1-dioxyde

Le produit attendu est obtenu selon le procédé décrit (temps de chauffe de 4 heures à 130 °C) dans le stade C de l'Exemple 1.
*Point de fusion : 166-168 °C*

### Stade C : 4-cyclopropyl-5,7,8-trifluoro-3,4-dihydro-2H-1,2,4-benzothiadiazine 1,1-dioxyde

Le produit attendu est obtenu selon le procédé décrit dans le stade D de l'Exemple 1.
*Point de fusion : 149-151 °C*

### EXEMPLE 68 : 7-chloro-4-cyclopropyl-6-fluoro-3,4-dihydro-2H-1,2,4-benzothiadiazine 1,1-dioxyde

### Stade A : 5-chloro-2-cyclopropylamino-4-fluorobenzènesulfonamide

Le produit attendu est obtenu à partir de la 5-chloro-2,4-difluorobenzènesulfonamide commerciale selon le procédé décrit dans le stade B de l'Exemple 1.

### Stade B : 7-chloro-4-cyclopropyl-6-fluoro-4H-1,2,4-benzothiadiazine 1,1-dioxyde

Le produit attendu est obtenu selon le procédé décrit (temps de chauffe de 5 heures à 130 °C) dans le stade C de l'Exemple 1.
*Point de fusion : 217-219 °C*

### Stade C : 7-chloro-4-cyclopropyl-6-fluoro-3,4-dihydro-2H-1,2,4-benzothiadiazine 1, 1-dioxyde

Le produit attendu est obtenu selon le procédé décrit dans le stade D de l'Exemple 1.
*Point de fusion :155-158 °C*

### EXEMPLE 69 : 7-aminocarbonyl-4-cyclopropyl-3,4-dihydro-2H-1,2,4-benzothiadiazine 1,1-dioxyde

### Stade A : 4-fluoro-3-sulfamoylbenzamide

La solution d'acide 4-fluoro-3-sulfamoylbenzoïque (5,4 mmol) dans le chlorure de thionyle (10 mL) est portée à reflux pendant 4 heures. Le réactif est ensuite éliminé par distillation sous pression réduite et le résidu est redissous dans le toluène sec (10 mL). Le solvant est éliminé par distillation sous pression réduite. Cette opération est renouvelée à deux reprises, puis le résidu est dissous dans le dioxanne sec (10 mL) et additionné d'ammoniaque (6 mmol) et de pyridine (6 mmol). Après une heure, le solvant est éliminé sous dépression et le résidu est repris par le méthanol (5 mL), puis additionné d'eau (30 mL). Le précipité obtenu est recueilli par filtration, lavé à l'eau et séché.
*Point de fusion : 212-214 °C*

### Stade B : 4-cyclopropylamino-3-sulfamoylbenzamide

Le produit attendu est obtenu selon le procédé décrit dans le stade B de l'Exemple 1.

### Stade C : 7-aminocarbonyl-4-cyclopropyl-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 278-281 °C*

### Stade D : 7-aminocarbonyl-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 269-2 71 °C*

### EXEMPLE 70 : 7-aminocarbonyl-4-cyclopropyl-3,4-dihydro-N-méthyl-2H-1,2,4-benzothiadiazine 1,1-dioxyde

### Stade A : 4-fluoro-N-méthyl-3-sulfamoylbenzamide

Le produit attendu est obtenu en utilisant la méthylamine à la place de l'ammoniaque selon le procédé décrit dans le stade A de l'Exemple 69.
*Point de fusion : 242-244 °C*

### Stade B : 4-cyclopropylamino-N-méthyl-3-sulfamoylbenzamide

Le produit attendu est obtenu selon le procédé décrit dans le stade B de l'Exemple 1.

### Stade C : 7-aminocarbonyl-4-cyclopropyl-N-méthyl-4H-1,2,4-benzothiadiazine 1,1-dioxyde

Le produit attendu est obtenu selon le procédé décrit (temps de chauffe de 3 heures à 130 °C) dans le stade C de l'Exemple 1.
*Point de fusion : 300-305 °C*

### Stade D : 7-aminocarbonyl-4-cyclopropyl-3,4-dihydro-N-méthyl-2H-1,2,4-benzothiadiazine 1,1-dioxyde

Le produit attendu est obtenu selon le procédé décrit dans le stade D de l'Exemple 1.
*Point de fusion : 271-273 °C*

### EXEMPLE 71 : 7-aminocarbonyl-4-cyclopropyl-3,4-dihydro-N,N-diméthyl-2H-1,2,4-benzothiadiazine 1,1-dioxyde

### Stade A : 4-fluoro-N,N-diméthyl-3-sulfamoylbenzamide

Le produit attendu est obtenu en utilisant la diméthylamine à la place de l'ammoniaque selon le procédé décrit dans le stade A de l'Exemple 69.

### Stade B : 4-cyclopropylamino-N,N-diméthyl-3-sulfamoylbenzamide

Le produit attendu est obtenu selon le procédé décrit dans le stade B de l'Exemple 1.

### Stade C : 7-aminocarbonyl-4-cyclopropyl-N,N-diméthyl-4H-1,2,4-benzothiadiazine 1, 1-dioxyde

Le produit attendu est obtenu selon le procédé décrit (temps de chauffe de 3 heures à 130 °C) dans le stade C de l'Exemple 1.
*Point de fusion :173-175 °C*

### Stade D : 7-aminocarbonyl-4-cyclopropyl-3,4-dihydro-N,N-diméthyl-2H-1,2,4-benzothiadiazine 1, 1-dioxyde

Le produit attendu est obtenu selon le procédé décrit dans le stade D de l'Exemple 1.
*Point de fusion : 216-219 °C*

### EXEMPLE 72 : 8-bromo-6-cyano-4-cyclopropyl-3,4-dihydro-2H-1,2,4-benzothiadiazine 1,1-dioxyde

### Stade A : 3-bromo-5-fluoro-4-sulfamoylbenzonitrile

Le produit attendu est obtenu à partir de la 2-bromo-4-cyano-5-fluoroaniline selon le procédé décrit dans le stade A de l'Exemple 1.

### Stade B : 3-bromo-5-cyclopropylamino-4-sulfamoylbenzonitrile

Le produit attendu est obtenu selon le procédé décrit dans le stade B de l'Exemple 1.

### Stade C : 8-bromo-6-cyano-4-cyclopropyl-4H-1,2,4-benzothiadiazine 1,1-dioxyde

Le produit attendu est obtenu selon le procédé décrit (temps de chauffe de 3 heures à 130 °C) dans le stade C de l'Exemple 1.

### Stade D : 8-bromo-6-cyano-4-cyclopropyl-3,4-dihydro-2H-1,2,4-benzothiadiazine 1,1-dioxyde

Le produit attendu est obtenu selon le procédé décrit dans le stade D de l'Exemple 1.
*Point de fusion : 250-253 °C*

### EXEMPLE 73 : 7-amino-4-cyclopropyl-3,4-dihydro-2H-1,2,4-benzothiadiazine 1,1-dioxyde

Le produit attendu est obtenu selon le procédé décrit dans le stade D de l'Exemple 1, à partir du 7-amino-4-cyclopropyl-4*H*-1,2,4-benzothiadiazine 1,1-dioxyde préparé au stade A de l'Exemple 42.
*Point de fusion : 18 7-189 °C*

### EXEMPLE 74 : 4-cyclopropyl-3,4-dihydro-6-nitro-2H-1,2,4-benzothiadiazine 1,1-dioxyde

### Stade A : 2-fluoro-4-nitrobenzènesulfonamide

Le produit attendu est obtenu à partir de la 2-fluoro-4-nitroaniline selon le procédé décrit dans le stade A de l'Exemple 41.
*Point de fusion : 145-147 °C*

### Stade B : 2-cyclopropylamino-4-nitrobenzènesulfonamide

Le produit attendu est obtenu selon le procédé décrit dans le stade B de l'Exemple 41.

### Stade C : 4-cyclopropyl-6-nitro-4H-1,2,4-benzothiadiazine 1,1-dioxyde

Le produit attendu est obtenu selon le procédé décrit dans le stade C de l'Exemple 41.
*Point de fusion : 254-25 7 °C*

### Stade D : 4-cyclopropyl-3,4-dihydro-6-nitro-2H-1,2,4-benzothiadiazine 1,1-dioxyde

Le produit attendu est obtenu selon le procédé décrit dans le stade D de l'Exemple 1.
*Point de fusion : 166-169 °C*

### EXEMPLE 75 : 6-acétamido-4-cyclopropyl-3,4-dihydro-2H-1,2,4-benzothiadiazine 1,1-dioxyde

### Stade A : 6-amino-4-cyclopropyl-4H-1,2,4-benzothiadiazine 1,1-dioxyde

Le produit attendu est obtenu à partir du composé de l'Exemple 74 selon le procédé décrit dans le stade A de l'Exemple 42.
*Point de fusion : 177-180 °C*

### Stade B : 6-acétamido-4-cyclopropyl-3,4-dihydro-2H-1,2,4-benzothiadiazine 1,1-dioxyde

Le produit attendu est obtenu selon le procédé décrit dans le stade B de l'Exemple 42.
*Point de fusion : 265-267 °C*

### EXEMPLE 76 : 6-amino-4-cyclopropyl-3,4-dihydro-2H-1,2,4-benzothiadiazine 1,1-dioxyde

Le produit attendu est obtenu selon le procédé décrit dans le stade D de l'Exemple 1, à partir du 6-amino-4-cyclopropyl-4*H*-1,2,4-benzothiadiazine 1,1-dioxyde préparé au stade A de l'Exemple 75.
*Point de fusion : 185-188 °C*

### EXEMPLE 77 : 4-cyclopropyl-3,4-dihydro-7-méthoxycarbonyl-2H-1,2,4-benzothiadiazine 1,1-dioxyde

### Stade A : Acide 4-fluoro-3-sulfamoylbenzoïque

La suspension de 2-fluoro-5-méthylbenzènesulfonamide (5,3 mmol), obtenu à partir de la 2-fluoro-5-méthylaniline selon le procédé décrit dans le stade A de l'Exemple 1, dans l'eau (50 mL) est chauffée à 70 °C puis additionnée goutte à goutte de NaOH 10 % m/v jusqu'à dissolution complète du composé. Le permanganate de potassium (3 g) est ajouté par petites portions. Après 4 heures sous agitation, la suspension est éliminée par filtration à chaud et le filtrat est amené à pH 1 par addition de HCl 12 N. Le précipité obtenu est recueilli par filtration, lavé à l'eau et séché pour donner le produit du titre sous la forme d'un solide blanc.
*Point de fusion : 23 7-239 °C*

### Stade B : 4-cyclopropylamino-3-sulfamoylbenzoate de méthyle

Préparé à partir du composé obtenu dans l'étape précédente selon le procédé décrit dans le stade B de l'Exemple 1, l'acide 4-cyclopropylamino-3-sulfamoylbenzoïque (3,9 mmol) est alors mis en solution dans l'acide sulfurique (0,5 mL) et le méthanol (10 mL) et le mélange est chauffé à reflux pendant 4 heures. Le solvant est ensuite éliminé par distillation sous pression réduite et le résidu est mis en suspension dans l'eau (20 mL). Le précipité obtenu est recueilli par filtration, lavé à l'eau et séché pour donner le produit du titre sous la forme d'un solide blanc.
*Point de fusion : 158-161 °C*

### Stade C : 4-cyclopropyl-7-méthoxycarbonyl-4H-1,2,4-benzothiadiazine 1,1-dioxyde

Le produit attendu est obtenu selon le procédé décrit (temps de chauffe de 4 h à 130 °C) dans le stade C de l'Exemple 1.
*Point de fusion : 192-195 °C*

### Stade D : 4-cyclopropyl-3,4-dihydro-7-méthoxycarbonyl-2H-1,2,4-benzothiadiazine 1,1-dioxyde

Le produit attendu est obtenu selon le procédé décrit dans le stade D de l'Exemple 1.
*Point de fusion : 178-180 °C*

### EXEMPLE 78 : 7-carboxy-4-cyclopropyl-3,4-dihydro-2H-1,2,4-benzothiadiazine 1,1-dioxyde

La solution du composé de l'Exemple 77 (3,5 mmol) dans un mélange méthanol/eau 1/1 (100 mL) contenant du NaOH (0,5 g) est chauffée à 40 °C pendant 3 heures. Le méthanol est ensuite éliminé par distillation sous pression réduite et la solution aqueuse résultante est ajustée à pH 2 par addition de HCl 6 N. Le précipité obtenu est recueilli par filtration, lavé à l'eau et séché pour donner le produit du titre sous la forme d'un solide blanc.
*Point de fusion : 263-265 °C*

### EXEMPLE 79 : 4-cyclopropyl-3,4-dihydro-7-phénoxycarbonyl-2H-1,2,4-benzothiadiazine 1,1-dioxyde

Le *N,N*'-carbonyldiimidazole (0,6 g) est additionné dans une solution du composé de l'Exemple 78 (3,5 mmol) dans le DMF (7 mL). Au bout de 1 heure d'agitation à température ambiante, le phénol (0,5 g) et le DBU (0,5 mL) sont ajoutés. Après 2 heures d'agitation à température ambiante, de l'eau (40 mL) est ajoutée et le milieu est extrait trois fois par de l'acétate d'éthyle (30 mL). Les phases organiques sont rassemblées, séchées sur MgSO₄ anhydre et filtrées. Le filtrat est évaporé à siccité et le résidu est redissous dans le méthanol (5 mL). La solution est additionné d'eau (50 mL) et le précipité obtenu est recueilli par filtration, lavé à l'eau et séché pour donner le produit du titre sous la forme d'un solide blanc.
*Point de fusion : 160-162 °C*

### EXEMPLE 80 : 4-cyclopropyl-3,4-dihydro-6-méthoxycarbonyl-2H-1,2,4-benzothiadiazine 1,1-dioxyde

### Stade A : 3-fluoro-4-sulfamoylbenzoate de méthyle

Une solution d'acide 3-fluoro-4-sulfamoylbenzoïque (4,5 mmol), obtenu en 2 étapes à partir de la 2-fluoro-4-méthylaniline selon le procédé décrit dans le stade A de l'Exemple 77, et d'acide sulfurique (0,5 mL) dans le méthanol (10 mL) est chauffée à reflux pendant 1 heure. Le solvant est ensuite éliminé par distillation sous pression réduite et le résidu est mis en suspension dans l'eau (20 mL). Le précipité obtenu est recueilli par filtration, lavé à l'eau et séché pour donner le produit du titre sous la forme d'un solide blanc.
*Point de fusion : 153-156 °C*

### Stade B : 3-cyclopropylamino-4-sulfamoylbenzoate de méthyle

Le produit attendu est obtenu selon le procédé décrit dans le stade B de l'Exemple 1.

### Stade C : 4-cyclopropyl-6-méthoxycarbonyl-4H-1,2,4-benzothiadiazine 1,1-dioxyde

Le produit attendu est obtenu selon le procédé décrit (temps de chauffe de 4 heures à 130 °C) dans le stade C de l'Exemple 1.
*Point de fusion : 191-194 °C*

### Stade D : 4-cyclopropyl-3,4-dihydro-6-méthoxycarbonyl-2H-1,2,4-benzothiadiazine 1,1-dioxyde

Le produit attendu est obtenu selon le procédé décrit dans le stade D de l'Exemple 1.
*Point de fusion : 162-164 °C*

### EXEMPLE 81 : 6-carboxy-4-cyclopropyl-3,4-dihydro-2H-1,2,4-benzothiadiazine 1,1-dioxyde

Le produit attendu est obtenu selon le procédé décrit dans l'Exemple 78, en utilisant comme produit de départ le composé de l'Exemple 80 à la place du composé de l'Exemple 77.
*Point de fusion : 243-245 °C*

### EXEMPLE 82 : 4-cyclopropyl-3,4-dihydro-7-phénylaminocarbonyl-2H-1,2,4-benzothiadiazine 1,1-dioxyde

### Stade A : 4-fluoro-N-phényl-3-sulfamoylbenzamide

Le produit attendu est obtenu en 4 étapes à partir du 4-fluoro-3-nitrotoluène.
Le 4-fluoro-3-nitrotoluène (6,5 mmol) est ajouté par petites portions à une solution de bichromate potassique (4 g) dans l'acide acétique glacial (10 mL). Après 15 minutes d'agitation, l'acide sulfurique (4 mL) est ajouté et la solution est portée à reflux pendant 2 heures. Le milieu est ensuite refroidi puis additionné d'eau (100 mL) et extrait par l'acétate d'éthyle (3 x 100 mL). Les phases organiques sont rassemblées, séchées sur MgSO₄ anhydre et évaporées. Le résidu est recristallisé dans un mélange acétate d'éthyle/hexane (1/1) est engagé dans l'étape suivante.
La solution d'acide 4-fluoro-3-nitrobenzoïque (5,4 mmol) dans le chlorure de thionyle (10 mL) est portée à reflux pendant 4 heures. Après évaporation du solvant sous dépression, le résidu est redissous dans le toluène sec (10 mL) et la solution est évaporée à siccité. Cette opération est répétée à deux reprises. Le résidu est dissous dans le dioxanne sec (10 mL) et la solution est additionnée d'aniline (6 mmol) et de pyridine (6 mmol). Après 1 heure d'agitation à température ambiante, le solvant est éliminé sous dépression et le résidu est dissous dans le méthanol (5 mL). Cette solution est additionnée d'eau (30 mL) et le précipité obtenu est recueilli par filtration, lavé à l'eau et séché pour être engagé dans l'étape suivante.
La suspension de 4-fluoro-3-nitro-*N*-phénylbenzamide (4 mmol) dans un mélange éthanol/eau 2/1 (30 mL) est portée à reflux puis additionnée de chlorure ammonique (0,5 g) et de poudre de fer (2 g). Après 15 minutes, la suspension est filtrée à chaud et l'insoluble est rincé à l'aide d'éthanol chaud (50 mL). Le filtrat est traité au charbon adsorbant et filtré. L'éthanol du filtrat est éliminé par distillation sous dépression. Le précipité obtenu est recueilli par filtration, lavé à l'eau et séché.
Le produit ainsi obtenu est mis en réaction selon le procédé décrit dans le stade A de l'Exemple 1 pour donner le produit du titre.
*Point de fusion : 219-221 °C*

### Stade B : 4-cyclopropylamino-N-phényl-3-sulfamoylbenzamide

Le produit attendu est obtenu selon le procédé décrit dans le stade B de l'Exemple 1.

### Stade C : 4-cyclopropyl-7-phénylaminocarbonyl-4H-1,2,4-benzothiadiazine 1,1-dioxyde

Le produit attendu est obtenu selon le procédé décrit (temps de chauffe de 4 heures à 130 °C) dans le stade C de l'Exemple 1.
*Point de fusion : 293-296 °C*

### Stade D : 4-cyclopropyl-3,4-dihydro-7-phénylaminocarbonyl-2H-1,2,4-benzothiadiazine 1,1-dioxyde

Le produit attendu est obtenu selon le procédé décrit dans le stade D de l'Exemple 1.
*Point de fusion : 268-270 °C*

### EXEMPLE 83 : 4-cyclopropyl-3,4-dihydro-6-phénylaminocarbonyl-2H-1,2,4-benzothiadiazine 1,1-dioxyde

### Stade A : 3-fluoro-N-phényl-4-sulfamoylbenzamide

Le produit attendu est obtenu à partir du 3-fluoro-4-nitrotoluène selon le procédé décrit dans le stade A de l'Exemple 82.
*Point de fusion : 250-252 °C*

### Stade B : 3-cyclopropylamino-N-phényl-4-sulfamoylbenzamide

Le produit attendu est obtenu selon le procédé décrit dans le stade B de l'Exemple 1.

### Stade C : 4-cyclopropyl-6-phénylaminocarbonyl-4H-1,2,4-benzothiadiazine 1,1-dioxyde

Le produit attendu est obtenu selon le procédé décrit (temps de chauffe de 4 heures à 130 °C) dans le stade C de l'Exemple 1.
*Point de fusion : 278-281 °C*

### Stade D : 4-cyclopropyl-3,4-dihydro-6-phénylaminocarbonyl-2H-1,2,4-benzothiadiazine 1,1-dioxyde

Le produit attendu est obtenu selon le procédé décrit dans le stade D de l'Exemple 1.
*Point de fusion : 232-234 °C*

### EXEMPLE 84 : 4-cyclopropyl-3,4-dihydro-7-méthoxy-2H-1,2,4-benzothiadiazine 1,1-dioxyde

### Stade A : 2-cyclopropylamino-5-méthoxybenzènesulfonamide

La solution de 2-amino-5-méthoxybenzènesulfonamide (4,9 mmol) dans le méthanol (20 mL) est additionnée de (1-éthoxycyclopropyloxy)triméthylsilane (4 mL) et d'acide acétique glacial (4 mL) puis portée à reflux pendant 18 heures. Le milieu est ensuite évaporé à siccité sous dépression. L'huile obtenue est reprise par de l'eau (30 mL) et extraite au chloroforme (3 x 30 mL). Les phases organiques sont rassemblées et séchées sur MgSO₄ anhydre. Après filtration, le filtrat est évaporé à siccité et l'huile résiduelle est mis en solution dans le THF (50 mL). Le borohydrure de sodium (2 g) et l'éthérate de trifluorure de bore (2 mL) sont ajoutés puis le mélange est chauffé à reflux pendant 18 heures. Le solvant est ensuite éliminé par évaporation sous pression réduite et le résidu est repris par de l'eau (30 mL), ajusté à pH légèrement acide par ajout de HCl 6 N, puis extrait au chloroforme (3 x 30 mL). Les phases organiques sont rassemblées et séchées sur MgSO₄ anhydre. Après filtration, le filtrat est évaporé à siccité et le composé attendu est obtenu sous forme d'une huile directement engagée dans l'étape suivante.

### Stade B : 4-cyclopropyl-7-méthoxy-4H-1,2,4-benzothiadiazine 1,1-dioxyde

Le produit attendu est obtenu selon le procédé décrit dans le stade C de l'Exemple 1.
*Point de fusion : 216-219 °C*

### Stade C : 4-cyclopropyl-3,4-dihydro-7-méthoxy-2H-1,2,4-benzothiadiazine 1,1-dioxyde

Le produit attendu est obtenu selon le procédé décrit dans le stade D de l'Exemple 1.
*Point de fusion : 154-156 °C*

### EXEMPLE 85 : 4-cyclopropyl-3,4-dihydro-7-hydroxy-2H-1,2,4-benzothiadiazine 1,1-dioxyde

Une solution du composé de l'Exemple 84 (50 mg) dans le chloroforme (3 mL) est refroidie sur bain de glace, puis additionnée de tribromure de bore (0,15 mL). Après 20 heures d'agitation, le milieu est additionné d'eau (5 mL), puis concentré sous dépression, et ensuite extrait par l'acétate d'éthyle (3 x 20 mL). Les phases organiques sont rassemblées et séchées sur MgSO₄ anhydre. Après filtration, le filtrat est évaporé à siccité et le résidu est dissous dans l'acétate d'éthyle (1 mL) puis additionné d'hexane (5 mL). Le précipité obtenu est recueilli par filtration, lavé à l'hexane et séché pour donner le produit attendu sous la forme d'un solide blanc.
*Point de fusion : 187-189 °C*

### EXEMPLE 86 : 6-ehloro-4-cyclopropyl-3,4-dihydro-7-méthoxy-2H-1,2,4-benzothiadiazine 1,1-dioxyde

Le produit attendu est obtenu selon le procédé décrit dans les stades A, B et C de l'Exemple 84 en utilisant comme produit de départ le 2-amino-4-chloro-5-méthoxybenzènesulfonamide à la place du 2-amino-5-méthoxybenzènesulfonamide.
*Point de fusion : 188-190 °C*

### EXEMPLE 87 : 6-chloro-4-cyclopropyl-3,4-dihydro-7-hydroxy-2H-1,2,4-benzothiadiazine 1,1-dioxyde

Le produit attendu est obtenu selon le procédé décrit dans l'Exemple 85 en utilisant comme produit de départ le composé de l'Exemple 86 à la place du composé de l'Exemple 84.
*Point de fusion :* 213-214 °C

### EXEMPLE 88 : 6,7-dichloro-4-cyclopropylméthyl-3,4-dihydro-2H-1,2,4-benzothiadiazine 1,1-dioxyde

### Stade A : 6,7-dichloro-4H-1,2,4-benzothiadiazine 1,1-dioxyde

Le produit attendu est obtenu à partir de la 2-amino-4,5-dichlorobenzènesulfonamide selon le procédé décrit dans le stade A de l'Exemple 60.
*Point de fusion : 237-240 °C*

### Stade B : 6,7-dichloro-4-cyclopropylméthyl-4H-1,2,4-benzothiadiazine 1,1-dioxyde

Le produit attendu est obtenu selon le procédé décrit dans le stade B de l'Exemple 60.
*Point de fusion : 192-195* °C

### Stade C : 6,7-dichloro-4-cyclopropylméthyl-3,4-dihydro-2H-1,2,4-benzothiadiazine 1,1-dioxyde

Le produit attendu est obtenu selon le procédé décrit dans le stade C de l'Exemple 60.
*Point de fusion : 171-173 °C*

### EXEMPLE 89 : 5-chloro-4-cyclopropyl-8-fluoro-3,4-dihydro-2H-1,2,4-benzothiadiazine 1,1-dioxyde

### Stade A : 3-chloro-2,6-difluorobenzènesulfonamide

Le produit attendu est obtenu à partir de la 3-chloro-2,6-difluoroaniline selon le procédé décrit dans le stade A de l'Exemple 1.
*Point de fusion : 144-146 °C*

### Stade B : 3-chloro-2-cyclopropylamino-6-fluorobenzènesulfonamide

Le produit attendu est obtenu selon le procédé décrit dans le stade B de l'Exemple 1.

### Stade C : 5-chloro-4-cyclopropyl-8-fluoro-4H-1,2,4-benzothiadiazine 1,1-dioxyde

Le produit attendu est obtenu selon le procédé décrit (temps de chauffe de 4 heures à 130 °C) dans le stade C de l'Exemple 1.
*Point de fusion : 201-204 °C*

### Stade D : 5-chloro-4-cyclopropyl-8-fluoro-3,4-dihydro-2H-1,2,4-benzothiadiazine 1,1-dioxyde

Le produit attendu est obtenu selon le procédé décrit dans le stade D de l'Exemple 1.
*Point de fusion : 171-174 °C*

### EXEMPLE 90 : 4-cyclopropyl-6,7-difluoro-3,4-dihydro-2H-1,2,4-benzothiadiazine 1,1-dioxyde

Le produit attendu est obtenu selon le procédé décrit dans les stades A, B et C de l'Exemple 84 en utilisant comme produit de départ le 2-amino-4,5-difluoro benzènesulfonamide à la place du 2-amino-5-méthoxybenzènesulfonamide.
*Point de fusion : 146-148 °C*

### ETUDE PHARMACOLOGIQUE

### EXEMPLE A : Etude de l'effet des produits sur le courant ionique induit par l'AMPA sur des cultures primaires de neurones de rat.

Le test consiste en la mesure *in vitro*, par fluorescence, de la dépolarisation membranaire induite sur des neurones embryonnaires de rat en culture, par l'action conjointe de l'AMPA et du produit testé, en comparaison à l'action de l'AMPA seul. Les cellules du cerveau sont mises en culture et maintenues dans un incubateur de culture cellulaire pendant 18 jours. Après incubation, le milieu de culture est retiré et remplacé par du milieu de chargement en sonde de fluorescence pour mesure du potentiel membranaire (20 µl ; kit de potentiel de membrane de Molecular Devices) et laissées à température ambiante pendant 1 heure. La fluorescence de base des puits est lue (appareil FDSS de Hamamatsu), puis l'AMPA est injecté sur les cellules (20 µl ; gamme de concentration de 3 à 100 µM) et l'action de l'AMPA est mesurée en cinétique. Le produit testé est ensuite introduit dans les puits (20 µl; en gamme de concentration, croisée avec celle de l'AMPA) et l'action du produit est mesurée en cinétique. A l'issue de chacune des 2 périodes de mesure en cinétique, la valeur retenue pour chaque puits est la moyenne de la lecture sur les 15 dernières secondes de la période. On représente les courbes d'effet de l'AMPA aux différentes concentrations de produit. Pour chaque concentration de produit, la valeur retenue est l'aire sous la courbe d'AMPA à cette concentration et l'EC_{2X}, concentration de produit qui double le potentiel membranaire induit par l'AMPA, est calculée.

Les composés de l'invention potentialisent fortement les effets excitateurs de l'AMPA comme le montre le tableau ci-dessous pour quelques exemples de composés de la présente invention :

| | **EC_{2X} (µM)** |
|---|---|
| **Exemple 1** | 1,0 |
| **Exemple 2** | 5,0 |
| **Exemple 21** | 2,0 |
| **Exemple 34** | 1,8 |
| **Exemple 37** | 4,4 |
| **Exemple 39** | 4,8 |
| **Exemple 62** | 0,8 |

### EXEMPLE B : Reconnaissance d'objet chez la souris CD1

Le test de reconnaissance d'objet (Behav. Brain Res., 1988, 31, 47-59) est basé sur l'activité exploratoire spontanée de l'animal et possède les caractéristiques de la mémoire épisodique chez l'Homme. Sensible au vieillissement (Eur. J. Pharm. 1997, 325, 173-180), ainsi qu'aux dysfonctionnements cholinergiques (Pharm. Biochem. Behav., 1996, 53(2), 277-283), ce test de mémoire est fondé sur l'exploration différentielle de 2 objets de forme assez similaire, l'un familier, l'autre nouveau. La procédure expérimentale, adaptée à la souris CD1, comporte 3 phases qui se déroulent dans la même enceinte expérimentale. Pendant la 1^{ère} phase qui dure 40 minutes, les souris sont habituées à l'environnement. Pendant la 2^{e} phase, qui a lieu le lendemain, un objet est placé dans l'enceinte et la souris est libre de l'explorer. Lorsque cette exploration atteint une durée de 20 secondes, la souris est retirée de l'enceinte. Au cours de la 3^{e} phase (5 minutes), 24 heures plus tard, le même objet est présenté (il acquiert le statut d'objet « familier »), ainsi qu'un nouvel objet. La durée d'exploration, exprimée en secondes, de chacun des deux objets, est chronométrée. Les animaux témoins, ayant reçu préalablement le véhicule par voie orale 60 minutes avant chacune des 3 phases, explorent pendant une durée équivalente l'objet « familier » et l'objet « nouveau », ce qui signifie l'oubli de l'objet déjà présenté. Les animaux ayant reçu un composé facilitateur mnémocognitif, explorent de façon préférentielle l'objet nouveau, ce qui signifie le maintien du souvenir de l'objet déjà présenté.

Les résultats obtenus avec les composés de la présente invention montrent une exploration significativement plus importante de l'objet nouveau par rapport à l'objet familier aux doses de 1 et 3 mg/kg, PO, en doublant voire triplant la durée d'exploration, ce qui indique que les composés de l'invention améliorent la mémorisation de façon importante.

Par exemple, la différence d'exploration des deux objets au cours de la 3^{e} phase est comprise entre 5 et 10 secondes après administration du composé de l'Exemple 1 alors qu'elle est inférieure à 3 secondes après administration du véhicule.

### EXEMPLE C : Composition pharmaceutique

| | |
|---|---|
| Formule de préparation pour 1000 comprimés dosés à 10 mg en 6,7-dichloro-4-cyclopropyl-3,4-dihydro-2*H*-1,2,4-benzothiadiazine 1,1-dioxyde (Exemple 1) | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 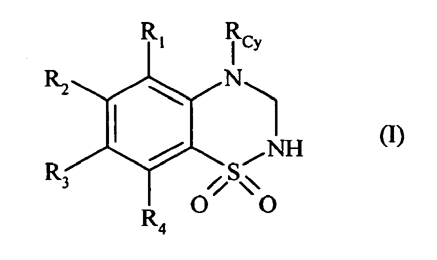 dans laquelle :
➢ R_{Cy} représente :
■ un groupement cycloalkyle (C₃-C₈) non-substitué ou substitué par un ou plusieurs groupements, identiques ou différents, choisis parmi alkyle (C₁-C₆) linéaire ou ramifié non-substitué ou substitué par un ou plusieurs atomes d'halogène ; alkoxy (C₁-C₆) linéaire ou ramifié ; hydroxy ; et amino non-substitué ou substitué par un ou deux groupements alkyle (C₁-C₆) linéaire ou ramifié ;
■ ou un groupement cycloalkyle (C₃-C₈) alkyle (C₁-C₆) linéaire ou ramifié non-substitué ou substitué sur la partie cyclique par un ou plusieurs groupements, identiques ou différents, choisis parmi alkyle (C₁-C₆) linéaire ou ramifié non-substitué ou substitué par un ou plusieurs atomes d'halogène ; alkoxy (C₁-C₆) linéaire ou ramifié ; hydroxy ; et amino non-substitué ou substitué par un ou deux groupements alkyle (C₁-C₆) linéaire ou ramifié ;
➢ R₁, R₂, R₃ et R₄, identiques ou différents, représentent chacun un atome d'hydrogène ou d'halogène ou un groupement nitro ; cyano ; hydroxy ; thio ; alkyle (C₁-C₆) linéaire ou ramifié non-substitué ou substitué par un ou plusieurs atomes d'halogène ; cyanoalkyle (C₁-C₆) linéaire ou ramifié ; hydroxyalkyle (C₁-C₆) linéaire ou ramifié ; alkoxy (C₁-C₆) linéaire ou ramifié non-substitué ou substitué par un ou plusieurs atomes d'halogène ; alkylthio (C₁-C₆) linéaire ou ramifié ; carboxy ; alkoxycarbonyle (C₁-C₆) linéaire ou ramifié ; aryloxycarbonyle ; acyle (C₁-C₆) linéaire ou ramifié ; amino non-substitué ou substitué par un ou deux groupements alkyle (C₁-C₆) linéaire ou ramifié ou par un groupement acyle (C₁-C₆) linéaire ou ramifié ; aminocarbonyle non-substitué ou substitué par un ou deux groupements alkyle (C₁-C₆) linéaire ou ramifié ; arylaminocarbonyle ; ou alkylsulfonylamino (C₁-C₆) linéaire ou ramifié ;
leurs énantiomères et leurs diastéréoisomères lorsqu'ils existent, ainsi que leurs sels d'addition avec un acide ou une base pharmaceutiquement acceptable,
on entend par « aryle », le groupement phényle non-substitué ou substitué par un ou plusieurs groupements, identiques ou différents, choisis parmi alkyle (C₁-C₆) linéaire ou ramifié non-substitué ou substitué par un ou plusieurs atomes d'halogène ; alkoxy (C₁-C₆) linéaire ou ramifié ; hydroxy ; et amino non-substitué ou substitué par un ou deux groupements alkyle (C₁-C₆) linéaire ou ramifié.

2. Composés de formule (I) selon la revendication 1, **caractérisés en ce que** R_{Cy} représente un groupement cycloalkyle (C₃-C₈).

3. Composés de formule (I) selon la revendication 1 ou 2, **caractérisés en ce que** R_{Cy} représente un groupement cyclopropyle.

4. Composés de formule (I) selon la revendication 1, **caractérisés en ce que** R_{Cy} représente un groupement cycloalkyle (C₃-C₈) alkyle (C₁-C₆) linéaire ou ramifié.

5. Composés de formule (I) selon la revendication 1 ou 4, **caractérisés en ce que** R_{Cy} représente un groupement cyclopropylméthyle.

6. Composés de formule (I) selon l'une quelconque des revendications 1 à 5, **caractérisés en ce que** R₁ représente un atome d'hydrogène ou un atome d'halogène.

7. Composés de formule (I) selon l'une quelconque des revendications 1 à 6, **caractérisés en ce que** R₂ représente un atome d'hydrogène, un atome d'halogène, un groupement méthyle, un groupement cyano ou un groupement carboxy.

8. Composés de formule (I) selon l'une quelconque des revendications 1 à 7, **caractérisés en ce que** R₃ représente un atome d'hydrogène, un atome d'halogène ou un groupement méthyle.

9. Composés de formule (I) selon l'une quelconque des revendications 1 à 8, **caractérisés en ce que** R₄ représente un atome d'hydrogène ou un atome d'halogène.

10. Composés de formule (I) selon l'une quelconque des revendications 1 à 9, **caractérisés en ce que** deux des groupements R₁, R₂, R₃ et R₄ sont différents d'un atome d'hydrogène.

11. Composés de formule (I) selon la revendication 1, **caractérisés en ce que** deux des groupements R₁, R₂, R₃ et R₄ représentent un atome d'halogène et R_{Cy} représente un groupement cyclopropyle.

12. Composés de formule (I) selon la revendication 1, qui sont le :
• 6,7-dichloro-4-cyclopropyl-3,4-dihydro-2*H*-1,2,4-benzothiadiazine 1,1-dioxyde ;
• 8-chloro-4-cyclopropyl-6-fluoro-3,4-dihydro-2*H*-1,2,4-benzothiadiazine 1,1-dioxyde ;
• 8-bromo-4-cyclopropyl-6-fluoro-3,4-dihydro-2*H*-1,2,4-benzothiadiazine 1,1-dioxyde ;
• 6-cyano-4-cyclopropyl-3,4-dihydro-2*H*-1,2,4-benzothiadiazine 1,1-dioxyde ;
• 8-bromo-6-chloro-4-cyclopropyl-3,4-dihydro-2*H*-1,2,4-benzothiadiazine 1,1-dioxyde ;
• 4-cyclopropyl-5-fluoro-3,4-dihydro-2*H*-1,2,4-benzothiadiazine 1,1-dioxyde ;
• 8-bromo-4-cyclopropyl-3,4-dihydro-2*H*-1,2,4-benzothiadiazine 1,1-dioxyde ;
• 4-cyclopropyl-5,7-difluoro-3,4-dihydro-2*H*-1,2,4-benzothiadiazine 1,1-dioxyde ;
• 6-fluoro-4-cyclopropyl-3,4-dihydro-2*H*-1,2,4-benzothiadiazine 1,1-dioxyde ;
• 8-chloro-4-cyclopropyl-7-fluoro-3,4-dihydro-2*H*-1,2,4-benzothiadiazine 1,1-dioxyde ;
• 4-cyclopropyl-3,4-dihydro-6-méthyl-2*H*-1,2,4-benzothiadiazine 1,1-dioxyde ;
• 4-cyclopropyl-8-fluoro-3,4-dihydro-2*H*-1,2,4-benzothiadiazine 1,1-dioxyde ;
• 8-chloro-4-cyclopropyl-3,4-dihydro-2*H*-1,2,4-benzothiadiazine 1,1-dioxyde ;
• 6-bromo-4-cyclopropyl-7-fluoro-3,4-dihydro-2*H*-1,2,4-benzothiadiazine 1,1-dioxyde ;
• 6-bromo-4-cyclopropyl-3,4-dihydro-2*H*-1,2,4-benzothiadiazine 1,1-dioxyde ;
• 4-cyclopropyl-6,8-difluoro-3,4-dihydro-2*H*-1,2,4-benzothiadiazine 1,1-dioxyde ;
• 4-cyclopropyl-5,6-difluoro-3,4-dihydro-2*H*-1,2,4-benzothiadiazine 1,1-dioxyde ;
• le 4-cyclopropyl-3,4-dihydro-7-méthyl-2*H*-1,2,4-benzothiadiazine 1,1-dioxyde ;
• le 6-chloro-4-cyclopropyl-3,4-dihydro-2*H*-1,2,4-benzothiadiazine 1,1-dioxyde ;
• le 4-cyclopropyl-3,4-dihydro-6-iodo-2*H*-1,2,4-benzothiadiazine 1,1-dioxyde ;
• le 6,7-dichloro-4-(1-méthyl)cyclopropyl-3,4-dihydro-2*H*-1,2,4-benzothiadiazine 1,1-dioxyde ;
• le 4-cyclopropylméthyl-7-fluoro-3,4-dihydro-2*H*-1,2,4-benzothiadiazine 1,1-dioxyde ;
• le 6-carboxy-4-cyclopropyl-3,4-dihydro-2*H*-1,2,4-benzothiadiazine 1,1-dioxyde ;
• le 4-cyclopropyl-7,8-difluoro-3,4-dihydro-2*H*-1,2,4-benzothiadiazine 1,1-dioxyde ;
• le 8-bromo-6-cyano-4-cyclopropyl-3,4-dihydro-2*H*-1,2,4-benzothiadiazine 1,1-dioxyde ;
• le 7-bromo-4-cyclopropyl-3,4-dihydro-2*H*-1,2,4-benzothiadiazine 1,1-dioxyde,
ainsi que leurs sels d'addition à un acide ou à une base pharmaceutiquement acceptable.

13. Procédé de préparation des composés de formule (I) selon la revendication 1, **caractérisé en ce que** l'on utilise comme produit de départ un composé de formule (II) : 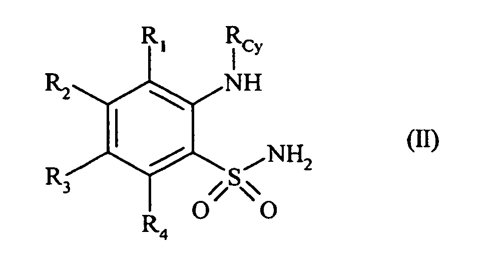 dans laquelle R_{Cy}, R₁, R₂, R₃ et R₄ sont tels que définis dans la revendication 1,
que l'on cyclise en présence d'un composé de formule (III) :
H-C(OR₅)₃ (III)
dans laquelle R₅ représente un groupement alkyle (C₁-C₆) linéaire ou ramifié,
pour conduire au composé de formule (IV) : 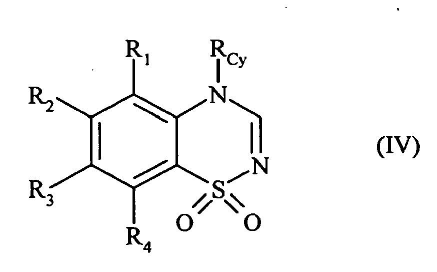 dans laquelle R_{Cy}, R₁, R₂, R₃ et R₄ sont tels que définis précédemment,
que l'on met en réaction avec un agent réducteur, pour conduire au composé de formule (I),
une variante dans la préparation des composés de formule (I) consistant, après réalisation de l'étape de réduction du composé de formule (IV), en l'utilisation des réactions classiques de chimie afin de modifier, dans un deuxième temps, les substituants du noyau benzénique,
composé de formule (I) qui peut être ensuite purifié selon une technique classique de séparation, que l'on transforme, si on le souhaite en ses sels d'addition à un acide ou à une base pharmaceutiquement acceptable et dont on sépare éventuellement les isomères, s'ils existent, selon une technique classique de séparation.

14. Procédé de préparation des composés de formule (I) selon la revendication 1, **caractérisé en ce que** l'on utilise comme produit de départ un composé de formule (V) : 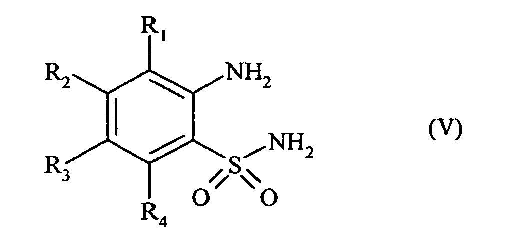 dans laquelle R₁, R₂, R₃ et R₄ sont tels que définis dans la revendication 1,
que l'on cyclise en présence d'un composé de formule (III) :
H-C(OR₅)₃ (III)
dans laquelle R₅ représente un groupement alkyle (C₁-C₆) linéaire ou ramifié,
pour conduire au composé de formule (VI) : 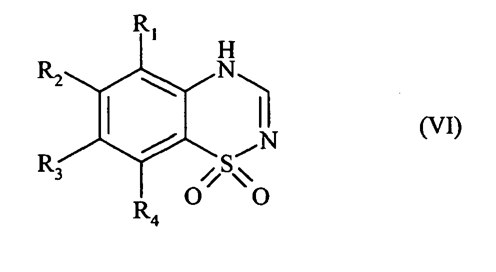 dans laquelle R₁, R₂, R₃ et R₄ sont tels que définis précédemment,
que l'on met en réaction avec un composé de formule (VII) :
Y-R_{Cy} (VII)
dans laquelle R_{Cy} est tel que défini précédemment et Y représente un groupement partant choisi parmi les atomes d'iode, de brome et les groupements tosylate, mésylate et triflate pour conduire au composé de formule (IV), 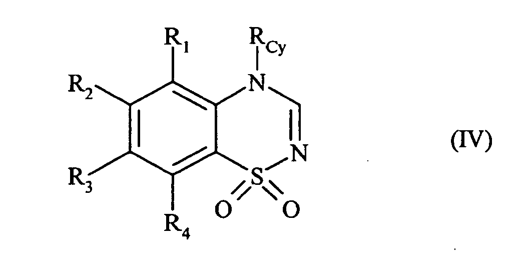 dans laquelle R_{Cy}, R₁, R₂, R₃ et R₄ sont tels que définis précédemment,
que l'on met en réaction avec un agent réducteur, pour conduire au composé de formule (I),
une variante dans la préparation des composés de formule (I) consistant, après réalisation de l'étape de réduction du composé de formule (IV), en l'utilisation des réactions classiques de chimie afin de modifier, dans un deuxième temps, les substituants du noyau benzénique,
composé de formule (I) qui peut être ensuite purifié selon une technique classique de séparation, que l'on transforme, si on le souhaite en ses sels d'addition à un acide ou à une base pharmaceutiquement acceptable et dont on sépare éventuellement les isomères, s'ils existent, selon une technique classique de séparation.

15. Composés de formule (IV) selon les revendications 13 ou 14 : 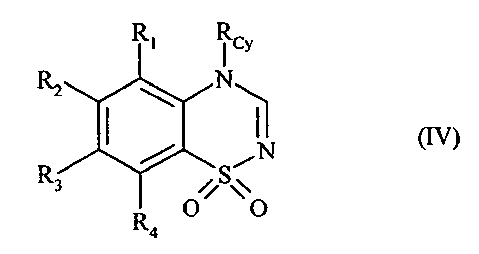 dans laquelle R_{Cy}, R₁, R₂, R₃ et R₄ sont tels que définis dans la revendication 1,
**caractérisés en ce qu'**ils sont utiles en tant qu'intermédiaires de synthèse des composés de formule (I).

16. Composition pharmaceutique contenant comme principe actif un composé selon l'une quelconque des revendications 1 à 12, en combinaison avec un ou plusieurs véhicules inertes, non toxiques et pharmaceutiquement acceptables.

17. Compositions pharmaceutiques selon la revendication 16 utiles en tant que modulateurs du récepteur AMPA.

18. Compositions pharmaceutiques selon la revendication 16 utiles pour le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

19. Utilisation des composés de formule (I) selon l'une quelconque des revendications 1 à 12 pour la fabrication de médicaments utiles en tant que modulateurs du récepteur AMPA.

20. Utilisation des composés de formule (I) selon l'une quelconque des revendications 1 à 12 pour la fabrication de médicaments utiles pour le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

## Claims

1. Compounds of formula (I): 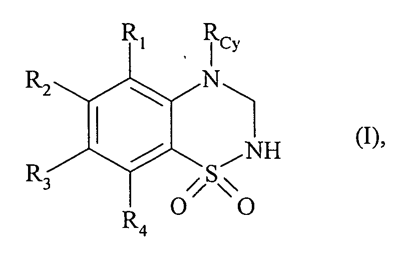 wherein:
➢ R_{Cy} represents:
■ a (C₃-C₈)cycloalkyl group which is unsubstituted or substituted by one or more identical or different groups selected from linear or branched (C₁-C₆)alkyl which is unsubstituted or substituted by one or more halogen atoms; linear or branched (C₁-C₆)alkoxy; hydroxy; and amino which is unsubstituted or substituted by one or two linear or branched (C₁-C₆)alkyl groups;
■ or a (C₃-C₈)cycloalkyl-(C₁-C₆)alkyl group in which the alkyl moiety is linear or branched and which is unsubstituted or substituted on the cyclic moiety by one or more identical or different groups selected from linear or branched (C₁-C₆)alkyl which is unsubstituted or substituted by one or more halogen atoms; linear or branched (C₁-C₆)alkoxy; hydroxy; and amino which is unsubstituted or substituted by one or two linear or branched (C₁-C₆)alkyl groups;
➢ R₁, R₂, R₃ and R₄, which may be the same or different, each represent a hydrogen or halogen atom or a nitro group; a cyano group; a hydroxy group; a thio group; a linear or branched (C₁-C₆)alkyl group which is unsubstituted or substituted by one or more halogen atoms; a linear or branched (C₁-C₆)cyanoalkyl group; a linear or branched (C₁-C₆)hydroxyalkyl group; a linear or branched (C₁-C₆)alkoxy group which is unsubstituted or substituted by one or more halogen atoms; a linear or branched (C₁-C₆)alkylthio group; a carboxy group; a linear or branched (C₁-C₆)-alkoxycarbonyl group; an aryloxycarbonyl group; a linear or branched (C₁-C₆)-acyl group; an amino group which is unsubstituted or substituted by one or two linear or branched (C₁-C₆)alkyl groups or by a linear or branched (C₁-C₆)acyl group; an aminocarbonyl group which is unsubstituted or substituted by one or two linear or branched (C₁-C₆)alkyl groups; an arylaminocarbonyl group; or a linear or branched (C₁-C₆)alkylsulphonylamino group;
their enantiomers and their diastereoisomers when they exist, and also addition salts thereof with a pharmaceutically acceptable acid or base,
"aryl" being understood to mean a phenyl group which is unsubstituted or substituted by one or more identical or different groups selected from linear or branched (C₁-C₆)alkyl which is unsubstituted or substituted by one or more halogen atoms; linear or branched (C₁-C₆)alkoxy; hydroxy; and amino which is unsubstituted or substituted by one or two linear or branched (C₁-C₆)alkyl groups.

2. Compounds of formula (I) according to claim 1, **characterised in that** R_{Cy} represents a (C₃-C₈)cycloalkyl group.

3. Compounds of formula (I) according to either claim 1 or claim 2, **characterised in that** R_{Cy} represents a cyclopropyl group.

4. Compounds of formula (I) according to claim 1, **characterised in that** R_{Cy} represents a (C₃-C₈)cycloalkyl-(C₁-C₆)alkyl group in which the alkyl moiety is linear or branched.

5. Compounds of formula (I) according to either claim 1 or claim 4, **characterised in that** R_{Cy} represents a cyclopropylmethyl group.

6. Compounds of formula (I) according to any one of claims 1 to 5, **characterised in that** R₁ represents a hydrogen atom or a halogen atom.

7. Compounds of formula (I) according to any one of claims 1 to 6, **characterised in that** R₂ represents a hydrogen atom, a halogen atom, a methyl group, a cyano group or a carboxy group.

8. Compounds of formula (I) according to any one of claims 1 to 7, **characterised in that** R₃ represents a hydrogen atom, a halogen atom or a methyl group.

9. Compounds of formula (I) according to any one of claims 1 to 8, **characterised in that** R₄ represents a hydrogen atom or a halogen atom.

10. Compounds of formula (I) according to any one of claims 1 to 9, **characterised in that** two of the groups R₁, R₂, R₃ and R₄ are other than a hydrogen atom.

11. Compounds of formula (I) according to claim 1, **characterised in that** two of the groups R₁, R₂, R₃ and R₄ represent a halogen atom and R_{Cy} represents a cyclopropyl group.

12. Compounds of formula (I) according to claim 1, which are:
• 6,7-dichloro-4-cyclopropyl-3,4-dihydro-2*H*-1,2,4-benzothiadiazine 1,1-dioxide;
• 8-chloro-4-cyclopropyl-6-fluoro-3,4-dihydro-2*H*-1,2,4-benzothiadiazine 1,1-dioxide;
• 8-bromo-4-cyclopropyl-6-fluoro-3,4-dihydro-2*H*-1,2,4-benzothiadiazine 1,1-dioxide;
• 6-cyano-4-cyclopropyl-3,4-dihydro-2*H*-1,2,4-benzothiadiazine 1,1-dioxide;
• 8-bromo-6-chloro-4-cyclopropyl-3,4-dihydro-2*H*-1,2,4-benzothiadiazine 1,1-dioxide;
• 4-cyclopropyl-5-fluoro-3,4-dihydro-2*H-*1,2,4-benzothiadiazine 1,1-dioxide;
• 8-bromo-4-cyclopropyl-3,4-dihydro-2*H*-1,2,4-benzothiadiazine 1,1-dioxide;
• 4-cyclopropyl-5,7-difluoro-3,4-dihydro-2*H*-1,2,4-benzothiadiazine 1,1-dioxide;
• 6-fluoro-4-cyclopropyl-3,4-dihydro-2*H*-1,2,4-benzothiadiazine 1,1-dioxide;
• 8-chloro-4-cyclopropyl-7-fluoro-3,4-dihydro-2*H*-1,2,4-benzothiadiazine 1,1-dioxide;
• 4-cyclopropyl-3,4-dihydro-6-methyl-2*H*-1,2,4-benzothiadiazine 1,1-dioxide;
• 4-cyclopropyl-8-fluoro-3,4-dihydro-2*H*-1,2,4-benzothiadiazine 1,1-dioxide;
• 8-chloro-4-cyclopropyl-3,4-dihydro-2*H*-1,2,4-benzothiadiazine 1,1-dioxide;
• 6-bromo-4-cyclopropyl-7-fluoro-3,4-dihydro-2*H*-1,2,4-benzothiadiazine 1,1-dioxide;
• 6-bromo-4-cyclopropyl-3,4-dihydro-2*H*-1,2,4-benzothiadiazine 1,1-dioxide;
• 4-cyclopropyl-6,8-difluoro-3,4-dihydro-2*H*-1,2,4-benzothiadiazine 1,1-dioxide;
• 4-cyclopropyl-5,6-difluoro-3,4-dihydro-2*H*-1,2,4-benzothiadiazine 1,1-dioxide;
• 4-cyclopropyl-3,4-dihydro-7-methyl-2*H*-1,2,4-benzothiadiazine 1,1-dioxide;
• 6-chloro-4-cyclopropyl-3,4-dihydro-2*H*-1,2,4-benzothiadiazine 1,1-dioxide;
• 4-cyclopropyl-3,4-dihydro-6-iodo-2*H*-1,2,4-benzothiadiazine 1,1-dioxide;
• 6,7-dichloro-4-(1-methyl)cyclopropyl-3,4-dihydro-2*H*-1,2,4-benzothiadiazine 1,1-dioxide;
• 4-cyclopropylmethyl-7-fluoro-3,4-dihydro-2*H*-1,2,4-benzothiadiazine 1,1-dioxide;
• 6-carboxy-4-cyclopropyl-3,4-dihydro-2*H*-1,2,4-benzothiadiazine 1,1-dioxide;
• 4-cyclopropyl-7,8-difluoro-3,4-dihydro-2*H*-1,2,4-benzothiadiazine 1,1-dioxide;
• 8-bromo-6-cyano-4-cyclopropyl-3,4-dihydro-2*H*-1,2,4-benzothiadiazine 1,1-dioxide;
• 7-bromo-4-cyclopropyl-3,4-dihydro-2*H*-1,2,4-benzothiadiazine 1,1-dioxide,
and also their addition salts with a pharmaceutically acceptable acid or base.

13. Process for the preparation of compounds of formula (I) according to claim 1, **characterised in that** there is used as starting material a compound of formula (II): 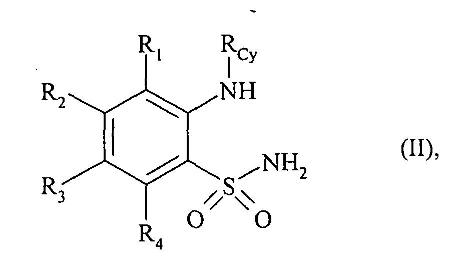 wherein R_{Cy}, R₁, R₂, R₃ and R₄ are as defined in claim 1,
which is cyclised in the presence of a compound of formula (III):
H-C(OR₅)₃ (III),
wherein R₅ represents a linear or branched (C₁-C₆)alkyl group,
to yield the compound of formula (IV): 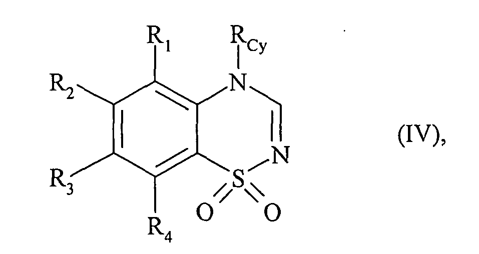 wherein R_{Cy}, R₁, R₂, R₃ and R₄ are as defined hereinbefore,
which is reacted with a reducing agent to yield the compound of formula (I),
a variant in the preparation of compounds of formula (I) consisting of using conventional chemical reactions after carrying out the reduction step on the compound of formula (IV) in order to subsequently change the substituents on the benzene ring, which compound of formula (I) may then be purified according to a conventional separation technique, is converted, if desired, into its addition salts with a pharmaceutically acceptable acid or base and is separated, where appropriate, into its isomers, if they exist, according to a conventional separation technique.

14. Process for the preparation of compounds of formula (I) according to claim 1, **characterised in that** there is used as starting material a compound of formula (V): 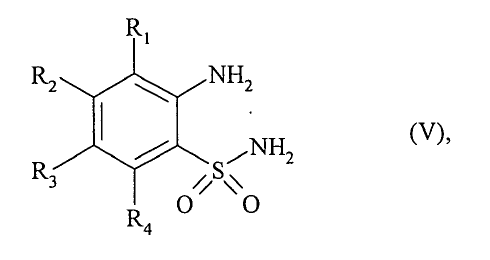 wherein R₁, R₂, R₃ and R₄ are as defined in claim 1,
which is cyclised in the presence of a compound of formula (III):
H-C(OR₅)₃ (III),
wherein R₅ represents a linear or branched (C₁-C₆)alkyl group,
to yield the compound of formula (VI): 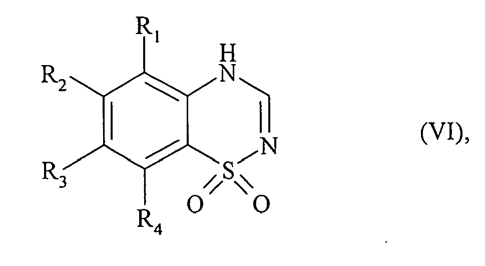 wherein R₁, R₂, R₃ and R₄ are as defined hereinbefore,
which is reacted with a compound of formula (VII):
Y-R_{Cy} (VII),
wherein R_{Cy} is as defined hereinbefore and Y represents a leaving group selected from iodine and bromine atoms and tosylate, mesylate and triflate groups to yield the compound of formula (IV): 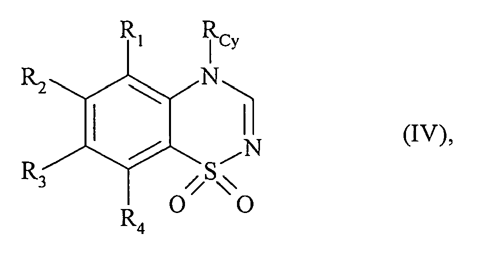 wherein R_{Cy}, R₁, R₂, R₃ and R₄ are as defined hereinbefore,
which is reacted with a reducing agent to yield the compound of formula (I),
a variant in the preparation of compounds of formula (I) consisting of using conventional chemical reactions after carrying out the reduction step on the compound of formula (IV) in order to subsequently change the substituents on the benzene ring,
which compound of formula (I) may then be purified according to a conventional separation technique, is converted, if desired, into its addition salts with a pharmaceutically acceptable acid or base and is separated, where appropriate, into its isomers, if they exist, according to a conventional separation technique.

15. Compounds of formula (IV) according to claims 13 or 14: 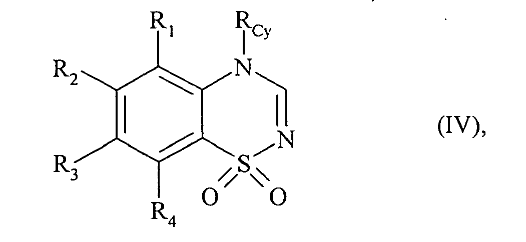 wherein R_{Cy}, R₁, R₂, R₃ and R₄ are as defined in claim 1,
**characterised in that** they are for use as synthesis intermediates for compounds of formula (I).

16. Pharmaceutical composition comprising as active ingredient a compound according to any one of claims 1 to 12 in combination with one or more inert, non-toxic, pharmaceutically acceptable carriers.

17. Pharmaceutical compositions according to claim 16 for use as modulators of the AMPA receptor.

18. Pharmaceutical compositions according to claim 16 for use in the treatment or prevention of disorders of memory and cognition that are associated with age, with syndromes of anxiety or depression, with progressive neurodegenerative diseases, with Alzheimer's disease, with Parkinson's disease, with Pick's disease, with Huntington's chorea, with Korsakoff's disease, with schizophrenia, with the sequelae of acute neurodegenerative diseases, with frontal lobe and subcortical dementias, with the sequelae of ischaemia and with the sequelae of epilepsy.

19. Use of compounds of formula (I) according to any one of claims 1 to 12 in the manufacture of medicaments for use as modulators of the AMPA receptor.

20. Use of compounds of formula (I) according to any one of claims 1 to 12 in the manufacture of medicaments for use in the treatment or prevention of disorders of memory and cognition that are associated with age, with syndromes of anxiety or depression, with progressive neurodegenerative diseases, with Alzheimer's disease, with Parkinson's disease, with Pick's disease, with Huntington's chorea, with Korsakoff disease, with schizophrenia, with the sequelae of acute neurodegenerative diseases, with frontal lobe and subcortical dementias, with the sequelae of ischaemia and with the sequelae of epilepsy.

## Patentansprüche

1. Verbindungen der Formel (I): 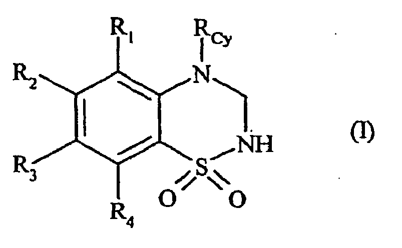 in der:
➢ R_{Cy}:
■ eine (C₃-C₈)-Cycloalkylgruppe, die nicht substituiert ist oder durch eine oder mehrere, identische oder verschiedenartige Gruppen substituiert ist, ausgewählt aus geradkettigem oder verzweigtem, nichtsubstituiertem oder durch ein oder mehrere Halogenatome substituiertem (C₁-C₆)-Alkyl; geradkettigem oder verzweigtem (C₁-C₆)-Alkoxy; Hydroxy; und nichtsubstituiertem oder durch eine oder zwei geradkettige oder verzweigte (C₁-C₆)-Alkylgruppen substituiertem Amino;
■ oder eine geradkettige oder verzweigte (C₃-C₈)-Cycloalkyl-(C₁-C₆)-alkylgruppe bedeutet, die nicht substituiert ist oder am cyclischen Teil durch eine oder mehrere, identische oder verschiedenartige Gruppen substituiert ist ausgewählt aus geradkettigem oder verzweigtem, nichtsubstituiertem oder durch ein oder mehrere Halogenatome substituiertem (C₁-C₆)-Alkyl; geradkettigem oder verzweigtem (C₁-C₆)-Alkoxy; Hydroxy; und nichtsubstituiertem oder durch eine oder zwei geradkettige oder verzweigte (C₁-C₆)-Alkylgruppen substituiertem Amino;
➢ R₁, R₂, R₃ und R₄, die identisch oder verschieden sind, jeweils ein Wasserstoffatom, ein Halogenatom oder eine Nitrogruppe; Cyanogruppe; Hydroxygruppe; Thiogruppe; geradkettige oder verzweigte, nichtsubstituierte oder durch ein oder mehrere Halogenatome substituierte (C₁-C₆)-Alkylgruppe; geradkettige oder verzweigte (C₁-C₆)-Cyanoalkylgruppe; geradkettige oder verzweigte (C₁-C₆)-Hydroxyalkylgruppe; geradkettige oder verzweigte, nichtsubstituierte oder durch ein oder mehrere Halogenatome substituierte (C₁-C₆)-Alkoxygruppe; geradkettige oder verzweigte (C₁-C₆)-Alkylthiogruppe; Carboxygruppe; geradkettige oder verzweigte (C₁-C₆)-Alkoxycarbonylgruppe; Aryloxycarbonylgruppe; geradkettige oder verzweigte (C₁-C₆)-Acylgruppe; nichtsubstituierte oder durch eine oder zwei geradkettige oder verzweigte (C₁-C₆)-Alkylgruppen oder durch eine geradkettige oder verzweigte (C₁-C₆)-Acylgruppe substituierte Aminogruppe; nichtsubstituierte oder durch eine oder zwei geradkettige oder verzweigte (C₁-C₆)-Alkylgruppen substituierte Aminocarbonylgruppe; Arylaminocarbonylgruppe oder geradkettige oder verzweigte (C₁-C₆)-Alkylsulfonylaminogruppe bedeuten;
deren Enantiomere und deren Diastereoisomere, wenn sie existieren, sowie ihre Additionssalze mit einer pharmazeutisch annehmbaren Säure oder Base,
wobei man unter «Aryl» die Phenylgruppe versteht, die nicht substituiert oder durch eine oder mehrere, identische oder verschiedenartige Gruppen substituiert ist ausgewählt aus geradkettigem oder verzweigtem, nichtsubstituiertem oder durch ein oder mehrere Halogenatome substituiertem (C₁-C₆)-Alkyl; geradkettigem oder verzweigtem (C₁-C₆)-Alkoxy; Hydroxy; und nichtsubstituiertem oder durch eine oder zwei geradkettige oder verzweigte (C₁-C₆)-Alkylgruppen substituiertem Amino.

2. Verbindungen der Formel (I) nach Anspruch 1, **dadurch gekennzeichnet, dass** R_{Cy} eine (C₃-C₈)-Cycloalkylgruppe bedeutet.

3. Verbindungen der Formel (I) nach Anspruch 1 oder 2, **dadurch gekennzeichnet, dass** R_{Cy} eine Cyclopropylgruppe bedeutet.

4. Verbindungen der Formel (I) nach Anspruch 1, **dadurch gekennzeichnet, dass** R_{Cy} eine geradkettige oder verzweigte (C₃-C₈)-Cycloalkyl-(C₁-C₆)-alkylgruppe bedeutet.

5. Verbindungen der Formel (I) nach Anspruch 1 oder 4, **dadurch gekennzeichnet, dass** R_{Cy} eine Cyclopropylmethylgruppe bedeutet.

6. Verbindungen der Formel (I) nach einem der Ansprüche 1 bis 5, **dadurch gekennzeichnet, dass** R₁ ein Wasserstoffatom oder ein Halogenatom bedeutet.

7. Verbindungen der Formel (I) nach einem der Ansprüche 1 bis 6, **dadurch gekennzeichnet, dass** R₂ ein Wasserstoffatom, ein Halogenatom, eine Methylgruppe, eine Cyanogruppe oder eine Carboxygruppe bedeutet.

8. Verbindungen der Formel (I) nach einem der Ansprüche 1 bis 7, **dadurch gekennzeichnet, dass** R₃ ein Wasserstoffatom, ein Halogenatom oder eine Methylgruppe bedeutet.

9. Verbindungen der Formel (I) nach einem der Ansprüche 1 bis 8, **dadurch gekennzeichnet, dass** R₄ ein Wasserstoffatom oder ein Halogenatom bedeutet.

10. Verbindungen der Formel (I) nach einem der Ansprüche 1 bis 9, **dadurch gekennzeichnet, dass** zwei der Gruppen R₁, R₂, R₃ und R₄ von einem Wasserstoffatom verschieden sind.

11. Verbindungen der Formel (I) nach Anspruch 1, **dadurch gekennzeichnet, dass** zwei der Gruppen R₁, R₂, R₃ und R₄ ein Halogenatom und R_{Cy} eine Cyclopropylgruppe bedeuten.

12. Verbindungen der Formel (I) nach Anspruch 1, nämlich:
• 6,7-Dichlor-4-cyclopropyl-3,4-dihydro-2*H*-1,2,4-benzothiadiazin-1,1-dioxid;
• 8-Chlor-4-cyclopropyl-6-fluor-3,4-dihydro-2*H*-1,2,4-benzothiadiazin-1,1-dioxid;
• 8-Brom-4-cyclopropyl-6-fluor-3,4-dihydro-2*H*-1,2,4-benzothiadiazin-1,1-dioxid;
• 6-Cyano-4-cyclopropyl-3,4-dihydro-2*H*-1,2,4-benzothiadiazin-1,1-dioxid;
• 8-Brom-6-chlor-4-cyclopropyl-3,4-dihydro-2*H*-1,2,4-benzothiadiazin-1,1-dioxid;
• 4-Cyclopropyl-5-fluor-3,4-dihydro-2*H*-1,2,4-benzothiadiazin-1,1-dioxid;
• 8-Brom-4-cyclopropyl-3,4-dihydro-2*H*-1,2,4-benzothiadiazin-1,1-dioxid;
• 4-Cyclopropyl-5,7-difluor-3,4-dihydro-2*H*-1,2,4-benzothiadiazin-1,1-dioxid;
• 6-Fluor-4-cyclopropyl-3,4-dihydro-2*H*-1,2,4-benzothiadiazin-1,1-dioxid;
• 8-Chlor-4-cyclopropyl-7-fluor-3,4-dihydro-2*H*-1,2,4-benzothiadiazin-1,1-dioxid;
• 4-Cyclopropyl-3,4-dihydro-6-methyl-2*H*-1,2,4-benzothiadiazin-1,1-dioxid;
• 4-Cyclopropyl-8-fluor-3,4-dihydro-2*H*-1,2,4-benzothiadiazin-1,1-dioxid;
• 8-Chlor-4-cyclopropyl-3,4-dihydro-2*H*-1,2,4-benzothiadiazin-1,1-dioxid;
• 6-Brom-4-cyclopropyl-7-fluor-3,4-dihydro-2*H*-1,2,4-benzothiadiazin-1,1-dioxid;
• 6-Brom-4-cyclopropyl-3,4-dihydro-2*H*-1,2,4-benzothiadiazin-1,1-dioxid;
• 4-Cyclopropyl-6,8-difluor-3,4-dihydro-2*H*-1,2,4-benzothiadiazin-1,1-dioxid;
• 4-Cyclopropyl-5,6-difluor-3,4-dihydro-2*H*-1,2,4-benzothiadiazin-1,1-dioxid;
• 4-Cyclopropyl-3,4-dihydro-7-methyl-2*H*-1,2,4-benzothiadiazin-1,1-dioxid;
• 6-Chlor-4-cyclopropyl-3,4-dihydro-2*H*-1,2,4-benzothiadiazin-1,1-dioxid;
• 4-Cyclopropyl-3,4-dihydro-6-iod-2*H*-1,2,4-benzothiadiazin-1,1-dioxid;
• 6,7-Dichlor-4-(1-methyl)-cyclopropyl-3,4-dihydro-2*H*-1,2,4-benzothiadiazin-1,1-dioxid;
• 4-Cyclopropylmethyl-7-fluor-3,4-dihydro-2*H*-1,2,4-benzothiadiazin-1,1-dioxid;
• 6-Carboxy-4-cyclopropyl-3,4-dihydro-2*H*-1,2,4-benzothiadiazin-1,1-dioxid;
• 4-Cyclopropyl-7,8-difluor-3,4-dihydro-2*H*-1,2,4-benzothiadiazin-1,1-dioxid;
• 8-Brom-6-cyano-4-cyclopropyl-3,4-dihydro-2*H*-1,2,4-benzothiadiazin-1,1-dioxid;
• 7-Brom-4-cyclopropyl-3,4-dihydro-2*H*-1,2,4-benzothiadiazin-1,1-dioxid,
sowie deren Additionssalze mit einer pharmazeutisch annehmbaren Säure oder Base.

13. Verfahren zur Herstellung der Verbindungen der Formel (I) nach Anspruch 1, **dadurch gekennzeichnet, dass** man als Ausgangsprodukt eine Verbindung der Formel (II) verwendet: 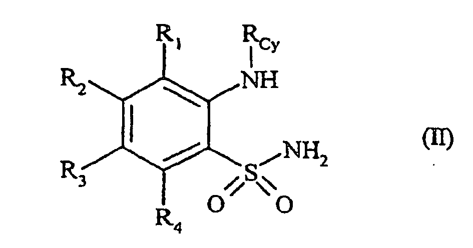 in der R_{Cy}, R₁, R₂, R₃ und R₄ die in Anspruch 1 angegebenen Bedeutungen besitzen, welche man in Gegenwart einer Verbindung der Formel (III):
H-C(OR₅)₃ (III)
in der R₅ eine geradkettige oder verzweigte (C₁-C₆)-Alkylgruppe darstellt, cyclisiert zur Bildung der Verbindung der Formel (IV): 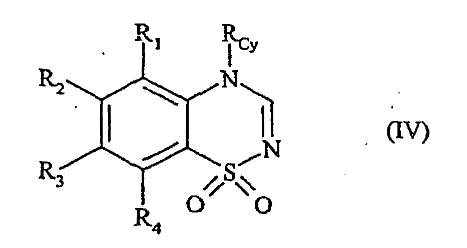 in der R_{Cy}, R₁, R₂, R₃ und R₄ die oben angegebenen Bedeutungen besitzen,
welche man mit einem Reduktionsmittel umsetzt zur Bildung der Verbindung der Formel (I),
wobei eine Variante der Herstellung der Verbindungen der Formel (I) darin besteht, dass man nach der Durchführung der Stufe der Reduktion der Verbindung der Formel (IV) in einer zweiten Stufe unter Anwendung von klassischen Reaktionen der Chemie die Substituenten des Benzolrings modifiziert,
welche Verbindung der Formel (I) anschließend mit Hilfe einer klassischen Trennmethode gereinigt werden kann, welche man gewünschtenfalls mit einer pharmazeutisch annehmbaren Säure oder Base in ihre Additionssalze überführt und welche man gegebenenfalls mit Hilfe einer klassischen Trennungstechnik in ihre Isomere, falls diese existieren, auftrennt.

14. Verfahren zur Herstellung der Verbindungen der Formel (I) nach Anspruch 1, **dadurch gekennzeichnet, dass** man als Ausgangsprodukt eine Verbindung der Formel (V) verwendet: 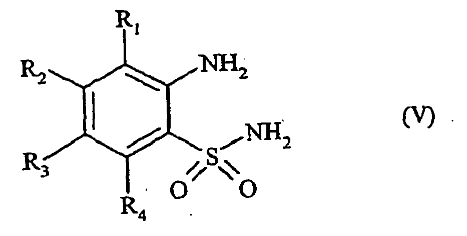 in der R₁, R₂, R₃ und R₄ die in Anspruch 1 angegebenen Bedeutungen besitzen, welche man in Gegenwart einer Verbindung der Formel (III):
H-C(OR₅)₃ (III)
in der R₅ eine geradkettige oder verzweigte (C₁-C₆)-Alkylgruppe darstellt, cyclisiert zur Bildung der Verbindung der Formel (VI): 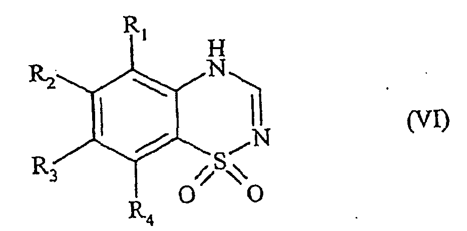 in der R₁, R₂, R₃ und R₄ die oben angegebenen Bedeutungen besitzen,
welche man mit einer Verbindung der Formel (VII) umsetzt:
Y-R_{Cy} (VII)
in der R_{Cy} die oben angegebenen Bedeutungen besitzt und Y eine austretende Gruppe ausgewählt aus Iod- und Bromatomen, Tosylat-, Mesylat- und Triflatgruppen darstellt, zur Bildung der Verbindung der Formel (IV): 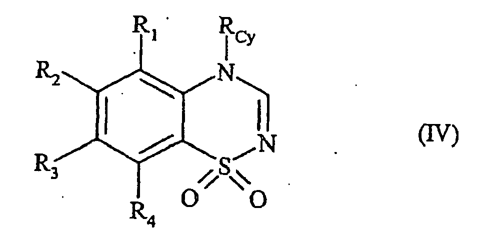 in der R_{Cy}, R₁, R₂, R₃ und R₄ die oben angegebenen Bedeutungen besitzen,
welche man mit einem Reduktionsmittel umsetzt zur Bildung der Verbindung der Formel (I),
wobei eine Variante der Herstellung der Verbindungen der Formel (I) darin besteht, dass man nach der Durchführung der Stufe der Reduktion der Verbindung der Formel (V) in einer zweiten Stufe unter Anwendung klassischer Reaktionen der Chemie die Substituenten des Benzolrings modifiziert,
welche Verbindung der Formel (I) anschließend mit Hilfe einer klassischen Trennmethode gereinigt werden kann, welche man gewünschtenfalls in ihre Additionssalze mit einer pharmazeutisch annehmbaren Säure oder Base überführt und die man gegebenenfalls mit Hilfe einer klassischen Trennmethode in ihre Isomeren, falls diese existieren, auftrennt.

15. Verbindungen der Formel (IV) nach den Ansprüchen 13 oder 14: 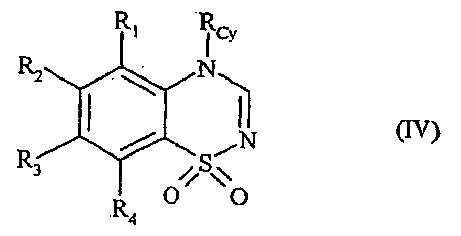 in der R_{Cy}, R₁, R₂, R₃ und R₄ die in Anspruch 1 angegebenen Bedeutungen besitzen, **dadurch gekennzeichnet, dass** sie nützlich sind als Zwischenprodukt zur Synthese der Verbindungen der Formel (I).

16. Pharmazeutische Zubereitung enthaltend als Wirkstoff eine Verbindung nach einem der Ansprüche 1 bis 12 in Kombination mit einem oder mehreren inerten, nichttoxischen und pharmazeutisch annehmbaren Hilfsstoffen.

17. Pharmazeutische Zubereitung nach Anspruch 16, nützlich als Modulatoren des Rezeptors AMPA.

18. Pharmazeutische Zubereitung nach Anspruch 16, nützlich zur Behandlung oder zur Vorbeugung von mnemokognitiven Störungen, die mit dem Alter verknüpft sind, Angst-oder Depressionssyndromen, progressiven neurodegenerativen Erkrankungen, der Alzheimerschen Krankheit, der Parkinsonschen Krankheit, der Pickschen Krankheit, der Huntington-Chorea, der Korsakoff-Krankheit, der Schizophrenie, den Folgen von akuten neurodegenerativen Erkrankungen, frontalen und subkortikalen Demenzen, Folgen von Ischämie und Folgen der Epilepsie.

19. Verwendung der Verbindungen der Formel (I) nach einem der Ansprüche 1 bis 12 für die Herstellung von Arzneimitteln, die nützlich sind als Modulatoren des Rezeptors AMPA.

20. Verwendung der Verbindungen der Formel (I) nach einem der Ansprüche 1 bis 12 für die Herstellung von Arzneimitteln, die nützlich sind zur Behandlung und zur Vorbeugung von mnemokognitiven Störungen, die mit dem Alter verknüpft sind, Angst- und Depressionssyndromen, progressiven neurodegenerativen Erkrankungen, der Alzheimerschen Krankheit, der Parkinsonschen Krankheit, der Pickschen Krankheit, der Huntington-Chorea, der Korsakoff-Krankheit, der Schizophrenie, den Folgen von akuten neurodegenerativen Erkrankungen, frontalen und subkortikalen Demenzen, Folgen von Ischämie und Folgen der Epilepsie.
